(19) Europäisches Patentamt
European Patent Office
Office européen des brevets

(11) Publication number: **0 125 702**
**B1**

(12) **EUROPEAN PATENT SPECIFICATION**

(45) Date of publication of patent specification: **12.08.87**

(21) Application number: **84105585.8**

(22) Date of filing: **16.05.84**

(51) Int. Cl.[4]: **C 07 C 91/10,** C 07 C 93/04, C 07 C 93/14, C 07 C 91/14, C 07 C 89/00, C 07 C 47/575, A 61 K 31/13, A 61 K 31/645, A 61 K 31/12, C 07 C 119/10, C 07 C 103/78

(54) **Polycyclic aromatic compounds.**

(30) Priority: **17.05.83 GB 8313571**

(43) Date of publication of application: **21.11.84 Bulletin 84/47**

(45) Publication of the grant of the patent: **12.08.87 Bulletin 87/33**

(84) Designated Contracting States: **AT BE CH DE FR GB IT LI LU NL SE**

(56) References cited:
**CH-A- 135 932**
**DE-A-2 362 749**
**SU-A- 182 132**
**US-A-2 865 925**
**US-A-4 211 726**

**ARZNEIMITTELFORSCHUNG, DRUG RESEARCH, vol. 7, 1982, Aulendorf HRABOWSKA et al. "Antitumor activity of 1-nitro-9-amino-acridine derivatives" pages 1013-1016**

**CHEMICAL ABSTRACTS, vol. 55, no. 4, February 20, 1961, Columbus, Ohio, USA L. NEDELEC et al. "10-Formylanthrone and its tautomeric enolic forms, (hydroxymethylene)anthrone and 9-hydroxy-10-anthraldehyde" column 3534, abstract no. 3 535a**

(73) Proprietor: **THE WELLCOME FOUNDATION LIMITED**
**183-193 Euston Road**
**London NW1 2BP (GB)**

(72) Inventor: **Bair, Kenneth Walter**
**342 Wesley Drive**
**Chapel Hill North Carolina 27514 (US)**

(74) Representative: **Sandmair, Kurt, Dr. et al**
**Patentanwälte Schwabe, Sandmair, Marx**
**Stuntzstrasse 16**
**D-8000 München 80 (DE)**

(56) References cited:
**CHEMICAL ABSTRACTS, vol. 56, no. 7, April 2, 1962, Columbus, Ohio, USA R. KUHN et al. "Cumulenes. XIII. Amalgam-hydrogenation of cumulenes and prototropic rearrangements", column 7180, abstract no. 7 181c**

**CHEMICAL ABSTRACTS, vol. 66, no. 11, March 13, 1967, Columbus, Ohio, USA D.W. CAMERON et al. "Some acyl derivatives of 2,6-dimethoxy-anthracene" page 4379, abstract no. 46 242w**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 125 702 B1**

Courier Press, Leamington Spa, England.

(56) References cited:

CHEMICAL ABSTRACTS, vol. 70, no. 9, March 3, 1969, Columbus, Ohio, USA N.P. BUU-HOI et al. "Novel synthesis of 6-methylchrysene and 6,12-dimethylchrysene: note on methylcoronene" page 318, abstract no. 37 532w

CHEMICAL ABSTRACTS, vol. 73, no. 17, October 26, 1970, Columbus, Ohio, USA N. CAMPBELL "3-Formylfluoranthene and 3,9-diacetylfluoroanthene" page 337, abstract no. 67 683d

REGISTRY HANDBOOK, 1974, supplement, Registry Numbers 43 226-08-0 through 51 771-93-4 CHEMICAL ABSTRACTS SERVICE, Columbus, Ohio, USA

REGISTRY HANDBOOK, 1981, supplement, Registry Numbers 78 198-84-2 through 80 386-99-8 CHEMICAL ABSTRACTS SERVICE, Columbus, Ohio, USA

BEILSTEINS "Handbuch der organischen Chemie", 4th edition, vol. 4, 1922 JULIUS SPRINGER, Berlin pages 303, 304

BEILSTEINS "Handbuch der organischen Chemie", 4th edition, vol. 4, supplement 3, 1962 SPRINGER-VERLAG, Berlin, Göttingen, Heidelberg pages 850-852

CHEMICAL ABSTRACTS, vol. 98, no. 13, March 28, 1983, Columbus, Ohio, USA G. CARDILLO et al. "A new synthesis of aminodiols and hydroxyaziridines using acetate and carbonate ions on a polymeric support" page 554, abstract no. 106 748p

CHEMICAL ABSTRACTS, vol. 63, no. 7, September 27, 1965, Columbus, Ohio, USA T. SUAMI "Aminocyclitols. V. New synthesis of 2-amino-1,3-cyclhexanediol" column 8220, abstract no. 8 220d

**Description**

The present invention relates to polycyclic aromatic alkanol derivatives which have been found to have biocidal activity. More specifically the invention concerns aminoalkanol derivatives containing a polycarbocyclic aromatic ring system, methods for the synthesis thereof, pharmaceutical formulations thereof, and their use as biocidal agents, particularly antitumour agents.

*Gazz. Chim. Ital., 93,* 1118, (1963) describes the preparation of 2-phenylmethylamino-2-methyl-1,3-propanediol but no antitumour activity is disclosed for this compound. Two analogues of nitracrine (1-nitro-9-((3′-dimethylaminopropyl)amino)acridine) containing 2-amino-2-methyl-1,3-propanediol and tris(hydroxymethyl)methylamine groups are disclosed in *Arzneim Forsch Drug Res. 3211,* 1013, (1982) as having antitumour activity in murine screening systems.

There are a variety of documents which are considered to represent background art.

US—PS 2,865,925 discloses a two step process for preparation of N-(2-hydroxyethyl)-N-(lower-alkyl)-9-amino-methylanthracenes which are useful as antifibrillatory agents as well as intermediates in preparing adrenolytic agents. The described compounds differ in two respects from the subject matter of the present application in that the amino group has an additional alkyl substituent and the compounds contain only one hydroxy group.

US—PS 4,211,726 discloses the synthesis of substituted 9,10-anthracenedicarboxaldehydes and substituted 9,10-dihydro-9,10-anthracenedicarboxaldehydes which are useful intermediates in the preparation of antibacterial agents.

CH—A—135932 discloses a process for preparing anthracene-9-aldehydes which is useful as pharmaceutical compound.

In Chem. Abstracts 56 (1962) 87 81 10 the preparation of 9-bromo-10-formylanthracene is described.

In Chem. Abstracts 66 (1967) 46 24 2w some acyl derivatives of 2,6-dimethoxyanthracene are described.

In Chem. Abstracts 70 (1969) 37 532w the preparation of 6-methylchrysene and 6,12-dimethylchrysene is described.

In Chem. Abstracts 73 (1970) 78 683 a method for the preparation of 3-formylfluoranthene and 3,9-diacetylfluoroanthene is disclosed.

In Chem. Abstracts Registry Handbook Supplement 1981, registry number 79075—24—4 the compound triphenylenecarboxaldehyde is identified and in Beilstein "Handbuch Der Organischen Chemie" 4. Auflage, Band 4, pages 303, 304 methods for the preparation of aminoderivatives of propanediol-(1,3) and 2-methylol-propanediol-(1,3) are disclosed.

In Beilstein "Handbuch Der Organischem Chemie", 4. Auflage, Band 4, III-Ergänzungswerk, page 850 methods for the preparation of 2-amino-2-ethyl-propanediol-(1.3) and 2-amino-1.3-dihydroxy-2-ethylpropane are disclosed. On page 852 a process for the preparation of aminoderivatives of 2-hydroxymethyl-pentanol-(1) is described.

In Chem. Abstracts 63 (1965) 8220d the synthesis of 2-amino-1,3-cyclohexanediol is disclosed.

We have now discovered a class of novel polycarbocyclic aromatic alkanol derivatives which have biocidal activity. Accordingly, in a first aspect, the present invention provides a compound of the formula (I):

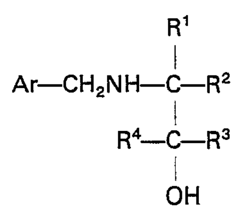

(I)

or a monomethyl or monoethyl ether thereof, the compound of formula (I) including its ethers containing no more than 28 carbon atoms in total, or an ester or salt thereof;
wherein Ar is selected from the group comprising:

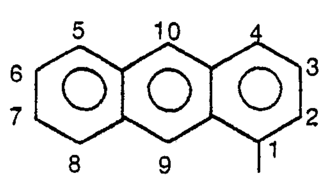
,
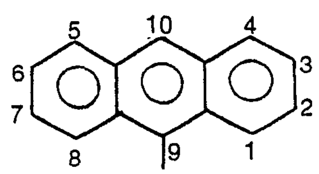
,
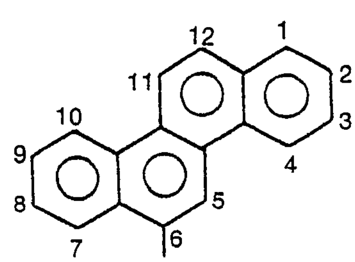
,

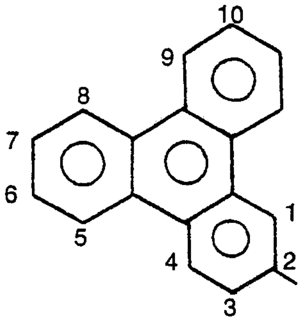 , 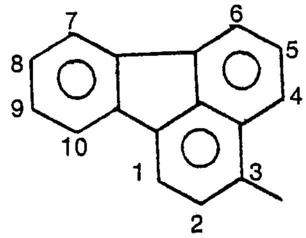 and 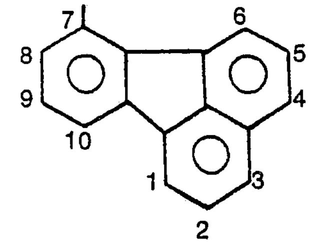

optionally substituted by one or two substituents which taken together contain not more than four carbon atoms in total and which are the same or different and are selected from halo; cyano; $C_{1-3}$ alkyl or $C_{1-3}$ alkoxy each optionally substituted by hydroxy or $C_{1-2}$ alkoxy; halo substituted $C_{1-2}$ alkyl or $C_{1-2}$ alkoxy; a group $S(O)_nR^5$ wherein n is an integer 0, 1 or 2 and $R^5$ is $C_{1-2}$ alkyl optionally substituted by hydroxy or $C_{1-2}$ alkoxy; or Ar is optionally substituted by a group $NR^6R^7$ containing not more than 5 carbon atoms wherein $R^6$ and $R^7$ are the same or different and each is a $C_{1-3}$ alkyl group or $NR^6R^7$ forms a five or six membered heterocyclic ring optionally containing one or two additional hetero atoms;

$R^1$ is $C_{1-3}$ alkyl substituted by hydroxy;

$R^2$ is hydrogen, $C_{1-3}$ alkyl or hydroxymethyl;

$R^3$ and $R^4$ are the same or different and each is hydrogen, methyl or ethyl;

$R^1$, $R^2$, $R^3$ and $R^4$ taken together containing not more than five carbon atoms; or the group:

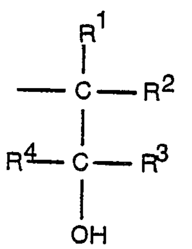 is 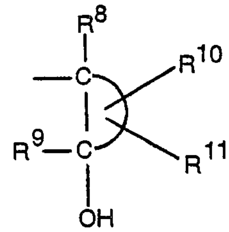

wherein 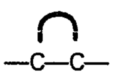

is a five or six membered saturated carbocyclic ring containing two or three hydroxy groups;

$R^8$ is hydrogen, methyl or hydroxymethyl;

$R^9$ and $R^{10}$ are the same or different and each is hydrogen or methyl;

$R^{11}$ is hydrogen, hydroxy, methyl or hydroxymethyl;

$R^8$, $R^9$, $R^{10}$, $R^{11}$ and the 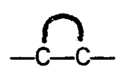

ring taken together containing less than seven carbon atoms.

Preferably, when Ar is 1- or 9- anthracenyl, the aromatic ring system is substituted.

Preferably Ar is 6-chrysenyl or 3- or 7-fluoranthenyl.

Particularly suitable substituents for the aromatic ring include $C_{1-2}$ alkyl or $C_{1-2}$ alkoxyl each optionally substituted by chloro, hydroxy or methoxy; or a group $S(O)_nR^5$ or chloro, imidazolyl, morpholino, cyano, bromo. Preferred substituents are chloro, 2-chloroethyl or $OCH_2CH_2R^{12}$ wherein $R^{12}$ is hydrogen, hydroxy or methoxy or a group $S(O)_nCH_3$ wherein n is the integer 0, 1 or 2. The substituents may be attached to any appropriate position on the aromatic ring. Preferably when Ar is substituted, this is by one substituent only.

Suitably

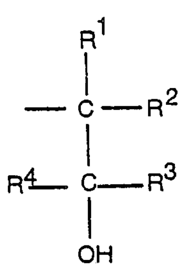 is 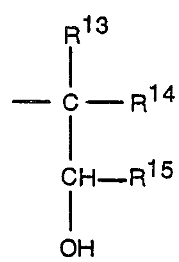 or 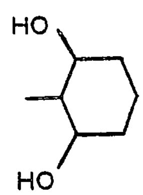

wherein

$R^{13}$ is $CH_2OH$, $CH(CH_3)OH$ or $CH_2CH_2OH$,

$R^{14}$ is hydrogen, $C_{1-3}$ alkyl, or $CH_2OH$

$R^{15}$ is hydrogen or methyl.

Preferably $R^{13}$ is $CH_2OH$ or $CH(CH_3)OH$. Suitably $R^{14}$ is hydrogen, methyl, ethyl or $CH_2OH$. Preferably the group:

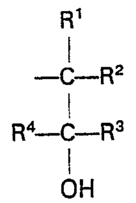 is 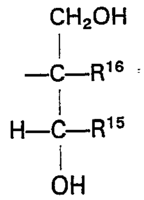

wherein $R^{15}$ is hydrogen or methyl and $R^{16}$ is hydrogen, methyl or ethyl, preferably methyl.

Salts included within the scope of the present invention are those of compounds of formula (I) and ethers and esters thereof.

Esters and non pharmaceutically acceptable salts of the compounds of formula (I) are useful intermediates in the preparation of compounds of the formula (I) and pharmaceutically acceptable salts thereof.

Thus, salts of the compounds of the formula (I) useful in the present invention include those derived from inorganic acids, such as hydrochloric, hydrobromic, sulfuric and phosphoric acids, and organic acids such as isethionic, maleic, malonic, succinic, salicyclic, tartaric, lactic, citric, formic, lactobionic and pantothenic acids; organic sulfonic acids, such as methanesulfonic, ethanesulfonic, benzenesulfonic, p-toluenesulfonic and naphthalene-2-sulfonic acids; ascorbic and amino acids, such as glycine. Suitable salts include hydrochlorides, methane and ethanesulfonates, lactates, citrates and isethionates. Pharmacologically and pharmaceutically acceptable salts are preferred, particularly those that are soluble in solvents suitable for parenteral administration, for example hydrochlorides, methanesulphonates and isethionates.

Esters of compounds of formula (I) are derived from acids known to those skilled in the art to be suitable for ester formation, and are conveniently those derived from $C_{1-6}$ alkanoic acids, for example acetic acid, propionic acid, n-butyric acid and iso-butyric acid.

The esters may be formed from all, or only some, of the hydroxy groups contained in the compounds of formula (I).

Specific compounds within the scope of formula (I) include, for example

2-((6-Chrysenylmethyl)amino)-2-methyl-1,3-propanediol,
2-((9-Anthracenylmethyl)amino)-2-methyl-1,3-propanediol,
2-((1-Anthracenylmethyl)amino)-2-methyl-1,3-propanediol,
2-((10-Chloro-9-anthracenylmethyl)amino-2-methyl-amino-1,3-propanediol,
2-(10-Bromo-9-anthracenylmethyl)amino)-2-methyl-1,3-propanediol,
2-Methyl-2-((10-methyl-9-anthracenylmethyl)amino)-1,3-propanediol,
2-Methyl-2-((10-methylthio-9-anthracenylmethyl)amino)-1,3-propanediol,
2-((10-(2-Chloroethyl)-9-anthracenylmethyl)amino)-2-methyl-1,3-propanediol,
2-((10-Hydroxymethyl)-9-anthracenylmethyl)amino)-2-methyl-1,3-propanediol,
10-((1,1-Bis)hydroxymethyl)ethylamino)methyl-9-anthracene-carbonitrile,
2-Methyl-2-((10-methylsulfinyl-9-anthracenylmethyl)amino)-1,3-propanediol,
2-((10-Methoxy-9-anthracenylmethyl)amino)-2-methyl-1,3-propanediol,
2-((10-Bromo-1-anthracenylmethyl)amino)-2-methyl-1,3-propanediol,
2-((4,10-Dichloro-9-anthracenylmethyl)amino)-2-methyl-1,3-propanediol,
2-((4,5-Dichloro-9-anthracenylmethyl)amino)-2-methyl-1,3-propanediol,
2-((2,10-Dichloro-9-anthracenylmethyl)amino)-2-methyl-1,3-propanediol,
2-((3,10-Dichloro-9-anthracenylmethyl)amino)-2-methyl-1,3-propanediol,
2-((3-Fluoranthrylmethyl)amino)-2-methyl-1,3-propanediol,
2-Methyl-2-((2-triphenylenylmethyl)amino)-1,3-propanediol,
2-((4-Chloro-9-anthracenylmethyl)amino)-2-methyl-1,3-propanediol,
2-((2-Chloro-9-anthracenylmethyl)amino)-2-methyl-1,3-propanediol,
2-((10-Ethylthio-9-anthracenylmethyl)amino)-2-methyl-1,3-propanediol,
2-((10-(2-Hydroxyethylthio)-9-anthracenylmethyl)amino)-2-methyl-1,3-propanediol,
2-((10-Chloro-9-anthracenylmethyl)amino)-2-hydroxymethyl-1,3-propanediol,
2-((7-Fluoranthenylmethyl)amino)-2-methyl-1,3-propanediol,
2-((10-(2-Hydroxyethyloxy)-9-anthracenylmethyl)amino-2-methyl-1,3-propanediol,
2-((10-Ethoxy-9-anthracenylmethyl)amino)-2-methyl-1,3-propanediol,
2-((6-Chrysenylmethyl)amino)-2-hydroxymethyl-1,3-propanediol,

2-((6-Chrysenylmethyl)amino)-2-ethyl-1,3-propanediol,
2-Hydroxymethyl-2-((3-fluoranthenylmethyl)amino)-1,3-propanediol,
2-Ethyl-2-((3-fluoranthenylmethyl)amino-1,3-propanediol,
2-((10-chloro-9-anthracenylmethyl)amino)-2-ethyl-1,3-propanediol,
2-((3-chloro-9-anthracenylmethyl)amino-2-methyl-1,3-propanediol,
(+−)(2R*,3S*)-2-((6-chrysenylmethyl)amino)-2-methyl-1,3-butanediol,
2-((2-ethyl-9-anthracenylmethyl)amino)-2-methyl-1,3-propanediol and
2-((3-ethyl-9-anthracenylmethyl)amino)-2-methyl-1,3-propanediol,
(+−)(2R*,3S*)-2-((9-anthracenylmethyl)amino)-2-methyl-1,3-butanediol,
(+−)(2R*,3R*)-2-(((6-chrysenyl)methyl)amino)-2-methyl-1,3-butanediol,
2-(((6-chrysenyl)methyl)amino)-2-ethoxymethyl-1,3-propanediol,
3-methoxy-2-(((6-chrysenyl)methyl)amino)-2-methyl-1-propanol,
3-methoxy-2-(((3-fluoranthenyl)methyl)amino)-2-methyl-1-propanol,
(+−)(2R*,2S*)-2-(((3-fluoranthenyl)methyl)amino)-2-methyl-1,3-butanediol,
2-ethoxymethyl-2-(((3-fluoranthenyl)methyl)amino)-1,3-propanediol,
2-(((9-anthracenyl)methyl)amino)-2-ethoxymethyl-1,3-propanediol,
2-β-(((6-chrysenylmethyl)amino)-1-α,3-α-cyclohexanediol,
2-β-((3-fluoranthenylmethyl)amino)-1-α,3-α-cyclohexanediol,
2-((6-chrysenylmethyl)amino)-2-isopropyl-1,3-propanediol,
2-((3-fluoranthenylmethyl)amino)-2-isopropyl-1,3-propanediol,
2-((6-chrysenylmethyl)amino)-2-methyl-1,4-butanediol,
2-((3-fluoranthenylmethyl)amino)-2-methyl-1,4-butanediol,
2-(((10-chloro-1-anthracenyl)methyl)amino)-2-methyl-1,3-propanediol,
Meso-3-((6-chrysenylmethyl)amino)-2,4-pentanediol,
2-((6-chrysenylmethyl)amino)-1,3-propanediol,
2-(((12-ethyl-6-chrysenyl)methyl)amino)-2-methyl-1,3-propanediol,
2-(((10-(2-methoxyethoxy)-9-anthracenyl)methyl)amino)-2-methyl-1,3-propanediol,
2-methyl-2-(((10-morpholino-9-anthracenyl)methyl)amino)-1,3-propanediol,
2-((9-anthracenylmethyl)amino)-3-methoxy-2-methyl-1-propanol,
2-(((12-chloro-6-chrysenyl)methyl)amino)-2-methyl-1,3-propanediol,
2-((9-anthracenylmethyl)amino)-2-isopropyl-1,3-propanediol,
2-((9)anthracenylmethyl)amino)-2-methyl-1,4-butanediol,
2-(((10-(1*H*-imidazol-1-yl)-9-anthracenyl)methyl)amino)-2-methyl-1,3-propanediol,
2-(4-ethyl-3-fluoranthenyl)methyl)amino)-2-methyl-1,3-propanediol,
2-(((12-ethoxy-6-chrysenyl)methyl)amino)-2-methyl-1,3-propanediol,
(1α, 2β, 3α)-2-(9-anthracenylmethyl)amino-1,3-cyclohexanediol,
2-(((4-chloro-10-hydroxyethoxy)-9-anthracenyl)methyl)amino)-2-methyl-1,3-propanediol,
(+−)(2R+, /RS+, 4R+)-3-(6-chrysenylmethyl)amino)-3-methyl-2, 5-pentanediol and
2-methyl-2-(((10-methylsulphonyl-1-9-anthracenyl)methyl)amino)-1,3-propanediol and salts and esters thereof.

Of these specific examples of compounds of formula (I), the most preferred compounds are 2-((6-chrysenylmethyl)amino)-2-methyl-1,3-propanediol, 2-((3-fluoranthenylmethyl)amino-2-methyl-1,3-propanediol, 2-((10-(2-hydroxyethyloxy)-9-(anthracenylmethyl)amino)-2-methyl-1,3-propanediol.

The compounds of formula (I) and their ethers, esters and salts thereof may be prepared by any method known in the art for the preparation of compounds of analogous structure. Thus the compounds of formula (I) may, for example, be prepared by any of the methods defined below.

1. The reduction of a compound of formula (II):

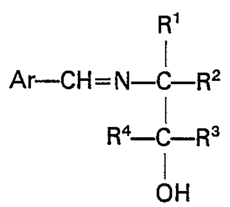

(II)

wherein $R^1$ to $R^4$ are as hereinbefore defined or an appropriately protected derivative thereof, followed by deprotection where appropriate. The conditions and reagents for such a reaction are well known to those skilled in the art and any such conditions/reagents may be employed. The reduction conveniently is carried out by a metal hydride such as lithium aluminium hydride, sodium borohydride or sodium cyanoborohydride, or by catalytic hydrogenation conveniently by hydrogen in the presence of a metal catalyst such as palladium or platinum or equivalent reagents as outlined by J. March, Advanced Organic Chemistry, 2nd ed., pages 819—820, McGraw Hill, New York, 1977. The reduction is suitably carried out with the compound of the formula (II) in solution in an inert solvent or mixture of solvents compatible with

the reducing agent at a non-extreme temperature, for example between 0° and 80°C and conveniently at room temperature.

In the case of lithium aluminium hydride and like reagents suitable solvents include ethers (for example tetrahydrofuran, diethylester and dimethoxyethane) optionally in the presence of a hydrocarbon co-solvent (for example toluene, benzene, or hexane).

In the case of sodium borohydride and like reagents, suitable solvents include alcohols (for example ethanol, methanol or isopropanol) optionally in the presence of a hydrocarbon co-solvent (for example toluene, benzene or hexane), or an ether co-solvent (for example diethylether or tetrahydrofuran).

In the case of sodium cyanoborohydride and like reagents, suitable solvents include those described for sodium borohydride and the reaction is suitably carried out in the presence of an acid conveniently glacial acetic acid as outlined in, for example, R. Hutchins et al, *Organic Preparations and Procedures International, 11,* 201, (1979).

In the case of catalytic hydrogenation, suitable solvents include alcohols (for example methanol and ethanol optionally in the presence of a hydrocarbon solvent (for example toluene or benzene) or an ether co-solvent (for example diethyl ether or tetrahydrofuran) in the presence of an acid (for example glacial acetic acid or ethanolic hydrochloride acid) or in glacial acetic acid.

Protected derivatives of compounds of formula (II) are conveniently used when lithium aluminium hydride is employed as the reducing agent. Convenient protecting groups are compatible with the reducing agent utilized and are readily removed under nondestructive conditions; for example benzyl, tetrahydropyranyl, and isopropylidene ethers.

It is often convenient not to isolate the compound of the formula (II) but to react a compound of the formula (III) with a compound of the formula (IV):

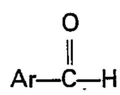

(III)

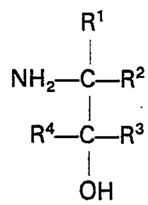

(IV)

wherein Ar and $R^1$ to $R^4$ are as hereinbefore defined, and reduce the compound of formula (II) so formed *in situ.* The reaction of the compounds of the formulae (III) and (IV) is again suitably carried out using conditions and reagents which are well known to those skilled in the art, for example in the presence of an acid, such as a sulfonic acid, i.e. p-toluenesulfonic acid, in an appropriate inert solvent, such as an aromatic hydrocarbon, for example toluene, with azeotropic removal of water followed by treatment with the reducing agent in an appropriate solvent, suitably ethanol or methanol. Alternatively, compounds of formula (II) formed under equilibrium conditions in appropriate solvents are reduced *in situ* with an appropriate reducing agent, suitably sodium cyanoborohydride. The compound of the formula (III) may be in the form of a protected aldehyde, for example an acetal, which liberates the aldehyde function under the reaction conditions.

In turn, a compound of formula (III) can be synthesised by reacting the appropriate polycyclic aromatic hydrocarbon with a formylating agent such as that generated by the reaction between $SnCl_4$ and $Cl_2CHOCH_3$ or equivalent reagents, for example, according to the method A. Reiche *et. al. Chem. Ber. 93,* 88 (1960), or with other standard formylating reagents/procedures known to the art, for example; the Gatterman-Koch reaction ($CO/HCl/AlCl_3/CuCl$), the Gatterman reaction ($HCN/HCl/ZnCl_2$), and the Vilsmeier reaction ($POCl_3$/PhN)Me)CHO, or $POCl_3/Me_2NCHO$) (J. March, *vide supra* pages 494—497.

The compounds of the formula (III) may also be prepared from an appropriate polycyclic aromatic hydrocarbon substituted by a suitable functional group such as $CH_2OH$, $CHBr_2$, N or methyl, and converting this functional group to an aldehyde group by methods well known to those skilled in the art.

Where the polycyclic aromatic ring bears *substituents,* the compound of formula (III) may be prepared by a variety of methods known in the art of organic chemistry depending on the nature of the substituent on the polycyclic ring. For example if the substituent(s) is a halogen, the starting materials may be prepared by direct treatment of the polycyclic aromatic hydrocarbon with a halogenating agent (e.g. $Cl_2$, $Br_2$, or $SO_2Cl_2$) or indirectly by such routes as the Sandmeyer reaction (D. T. Moury, *Chem. Rev. 42,* 213 (1948)). If the substituent(s) is alkyl, the polycyclic aromatic hydrocarbon may be reacted with the appropriate reagents under Friedel-Crafts reaction conditions (P. Gore, *Chem. Rev. 55* 229, (1955)).

The compounds of the formula (IV) also may be prepared by methods known in the art, for example by the reaction of compound $NO_2CH_2R^2$ with an appropriate aldehyde, conveniently acetaldehyde or formaldehyde, (as in B. M. Vanderbilt and H. B. Haas, *Ind. Eng. Chem. 32,* 34 (1940)) followed by reduction (as outlined in T. March, *vide supra* 1125—1126) conveniently by hydrogen in the presence of a metal

catalyst, for example a platinum containing catalyst, in an appropriate solvent, conveniently glacial acetic acid.

2). The reduction of a compound of the formula (V):

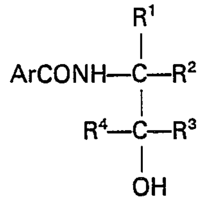

(V)

wherein Ar and $R^1$ to $R^4$ are as hereinbefore defined and the hydroxy groups are optionally protected, followed by deprotection of the hydroxy groups where appropriate. The reduction may be carried out by standard reducing agents known for carrying out this type of reduction, for example, a hydride such as lithium aluminium hydride in an inert solvent, such as an ether, i.e. tetrahydrofuran at a non-extreme temperature, for example, at between 0° and 100°C and conveniently at the reflux temperature of the ether.

The compound of the formula (V) may be formed by the reaction of the appropriate acid ArCOOH, or a suitable reactive acid derivative thereof, for example, an acid halide, in an inert solvent with an amine of the formula (IV) in which the hydroxy groups are optionally protected; for example when the compound of the formula (IV) is a diol, by an isopropyledene group. The compound of the formula (V) so formed is suitably reduced *in situ* and deprotected where appropriate to a compound of the formula (I). The compounds of the formula ArCOOH can be prepared by methods well known to those skilled in the art.

3) The reaction of a compound $ArCH_2L$, wherein Ar is as hereinbefore defined and L is a leaving group, with a compound of the formula (IV) as hereinbefore defined. Suitable leaving groups are those defined by J. March, *vide supra* pages 683 and 895, and include halogens, such as chlorine and bromine, and sulphonic acid derivatives such as p-toluenesulfonate. The reaction is suitably carried out in an appropriate solvent, such as a dipolar aprotic solvent or alcohol at a non-extreme temperature, for example between 50° and 150° conveniently between 50 and 100°. The compounds of the formula $ArCH_2L$ can be prepared by methods well known to those skilled in the art.

There is therefore provided, as a further aspect of the invention, a method for the preparation of a compound of formula (I) comprising any method known for the preparation of analogous compounds, in particular those methods defined in (1) to (3) hereinabove.

The compounds of formula (I) have biocidal activity in that they are toxic to certain living cells that are detrimental to mammals, for example, pathogenic organisms and tumour cells. This toxicity to pathogenic organisms has been demonstrated by activity against viruses (e.g., *Herpes simplex* 1/vero), bacteria (e.g., *Mycoplasma smegmatis* and *Streptococcus Pyogenes*) fungi (e.g., *Candida albicans*) protozoa (e.g., *Eimeria tenella*) and helminths (e.g., *Nippostrongylus brasiliensis*). The antitumour activity of compounds of formula (I) has been shown in a number of recognized screens and primarily by the activity against ascitic P388/0 leukaemia. The activity against ascitic tumours, including P388/0, is evidenced by reduction of tumour cell number in mammals, for example mice bearing ascitic tumours, and their consequent increase in survival duration as compared to an untreated tumour-bearing group. Antitumour activity is further evidenced by measurable reduction in the size of certain solid tumours following treatment of mice with the compounds of this invention compared to an untreated tumour-bearing control group. Thus, compounds of formula (I) have been shown to be active against murine tumours, lymphocytic leukaemia P388/0, lymphocytic leukaemia L1210, melanotic melanoma B16, P815 mastocytoma, MDAY/D2 fibrosarcoma, colon 38 adenocarcinoma, M5076 rhabdomyosarcoma, and Lewis lung carcinoma.

Activity in one or more of these tumour tests has been reported to be indicative of antitumour activity in man (A. Goldin *et. al* in Methods of Cancer Research ed. V. T. DeVita. Jr., and H. Busch, *16* 165, Academic Press, N.Y. 1979).

There are sublines of P388/0 which have been made resistant to the following clinically useful agents; cytosine arabinoside, doxorubicin, cyclophosphamide, L-phenylalanine mustard, methotrexate, 5-fluorouracil, actinomycin D, cis-platin, and bis-chloroethylnitrosourea. Compounds of formula (I) show potent activity against those drug-resistant tumours using the test procedure for P388/0 above.

Compounds of formula (I) have also been found to be active against human tumour cells in primary cultures of gastric, pancreatic, mesothelioma, myeloma, and colon cancers. (As used herein "cancer" is to be taken as synonymous with "malignant tumour" or more generally "tumour" unless otherwise noted). This is a procedure in which the prevention of tumour cell colony formation, *i.e.*, tumour cell replication, by a drug has been shown to correlate with clinical antitumour activity in man (D. D. Von Hoff et al, *Cancer Chemotherapy and Pharmacology 6,* 265 (1980); S. Salmon and D. D. Von Hoff, *Seminars in Oncology, 8,* 377 (1981).

Compounds of formula (I) which have been found to have antitumour activity intercalate *in-vitro* with DNA. This property is determined by viscometric methods using the procedure of W. D. Wilson *et al, Nucleic Acids Research 4,* 2697, (1954), and a log P as calculated by the method of C. Hansch and A. Leo in Substituent Constants for correlation analysis in Chemistry and Biology, John Wiley and Sons, New York

1979, lying in the range between −2 and +2.5.

There is further provided as a further, or alternative, aspect of the invention, a compound of formula (I) for use in therapy, for example as an antitumour agent.

The amount of compound of formula (I) required to be effective as a biocidal agent will, of course, vary and is ultimately at the discretion of the medical or veterinary practitioner. The factors to be considered include the condition being treated, the route of administration, the nature of the formulation; the mammal's body weight, surface area, age and general condition; and the particular compound to be administered. A suitable effective antitumour dose is in the range of about 0.1 to about 120 mg/kg bodyweight, preferably in the range of about 1.5 to 50 mg/kg for example 10 to 30 mg/kg. The total daily dose may be given as a single dose, multiple doses, *e.g.,* two to six times a day or by intravenous infusion for a selected duration. For example, with a 75 kg mammal, the dose range would be about 8 to 9000 mg per day, and a typical dose would be about 2000 mg per day. If discrete multiple doses are indicated, treatment might typically be 500 mg of a compound of formula I given 4 times per day in the form of a tablet, capsule, liquid (*e.g.,* syrup) or injection.

Whilst it is possible for the active compound (defined herein as compound of formula I or ether, ester or salt thereof) to be administered alone as the raw chemical it is preferable to present the active compound in a pharmaceutical formulation. Formulations of the present invention, for medical use, comprise an active compound together with one or more pharmaceutically acceptable carriers thereof and optionally other therapeutic ingredients. The carrier(s) must be pharmaceutically acceptable in the sense of being compatible with the other ingredients of the formulation and not deleterious to the recipient thereof.

The present invention therefor, further provides a pharmaceutical formulation comprising a compound of formula (I) (in the form of the free base, ether, ester or a pharmaceutically acceptable acid addition salt thereof) together with a pharmaceutically acceptable carrier therefor.

There is also provided a method for the preparation of a pharmaceutical formulation comprising bringing into association a compound of formula (I) or an ether, ester or a pharmaceutically acceptable salt thereof and a pharmaceutically acceptable carrier therefor.

Whilst the antitumour activity of the compounds of formula (I) is believed to reside in the free base, it is often convenient to administer an acid addition salt of a compound of the formula (I).

The formulations include those suitable for oral, rectal or parenteral (including subcutaneous, intramuscular and intravenous) administration.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into association with a liquid carrier or a finely divided solid carrier or both and then, if necessary, shaping the product into desired formulations.

Formulations of the present invention suitable for oral administration may be presented as discrete units such as capsules, cachets, tablets or lozenges, each containing a predetermined amount of the active compound; as a powder or granules; or a suspension in an aqueous liquid or non-aqueous liquid such as a syrup, an elixir, an emulsion or a draught.

A tablet may be made by compression or moulding, optionally with one or more accessory ingredients. Compressed tablets may be prepared by compressing in a suitable machine the active compound in a free-flowing form such as a powder or granules, optionally mixed with a binder, lubricant, inert diluent, surface active or dispersing agent. Moulded tablets may be made by moulding in a suitable machine, a mixture of the powdered active compound with any suitable carrier.

A syrup may be made by adding the active compound to a concentrated, aqueous solution of a sugar for example sucrose to which may also be added any accessory ingredient. Such accessory ingredient(s) may include flavourings, an agent to retard crystallisation of the sugar or an agent to increase the solubility of any other ingredient, such as a polyhydric alcohol for example glycerol or sorbitol.

Formulations for rectal administration may be presented as a suppository with a conventional carrier such as cocoa butter.

Formulations suitable for parenteral administration conveniently comprise a sterile aqueous preparation of the active compound which is preferably isotonic with the blood of the recipient. Such formulations suitably comprise a solution of a pharmaceutically and pharmacologically acceptable acid addition salt of a compound of the formula that is isotonic with the blood of the recipient. Thus, such formulations may conveniently contain distilled water, 5% dextrose in distilled water or saline and a pharmaceutically and pharmacologically acceptable acid addition salt of a compound of the formula (I) that has an appropriate solubility in these solvents, for example the isethionate and methane sulphonate salts and preferably the latter.

In addition to the aforementioned ingredients, the formulations of this invention may further include one or more accessory ingredient(s) selected from diluents, buffers, flavouring agents, binders, surface active agents, thickeners, lubricants, preservatives (including antioxidants) and the like.

The following Examples are provided by the way of illustration of the present invention and should in no way be construed as a limitation thereof. All temperatures indicated are in degrees Celsius.

General Comments

All solvents were reagent grade and used without further purification with the following exceptions. THF was dried by distillation from Na/K alloy under nitrogen ($N_2$) and used immediately. Toluene ($PhCH_3$) was distilled from $CaH_2$ under $N_2$ and stored over 3A molecular sieves. Chemicals used were reagent grade and used without purification unless noted. The full name and address of the suppliers of the reagents and chemicals is given when first cited. After this, an abbreviated name is used.

Preparative HPLC was carried out on a Waters Prep LC/System 500A machine using two 500 g silica gel ($SiO_2$) cartridges unless otherwise noted. Plugs of $SiO_2$ used for purifications were "flash chromatography" silica gel (E. Merck, silica gel 60, 230—400 mesh). An appropriate volume sintered glass funnel was filled approximately 3/4 full with the $SiO_2$ and packed evenly by tapping the outside of the funnel. A piece of filter paper was then placed on top of the $SiO_2$ and a solution of the material to be purified applied evenly on the top. Gentle suction through a filter flask moved the eluting solvent through the plug rapidly. The appropriate size fractions were combined as needed and further manipulated.

Satisfactory elemental analyses were obtained for all exemplified compounds of formula (I). Where elemental analyses were performed on the intermediates or starting materials, those elements analysed for are indicated eg (C, H, N) or (C, H, Cl) etc. In all of the abovementioned analyses, the experimentally determined values were within ±0.4% of the calculated values.

Preparation of Starting Materials

A. 6-Chrysencarbaldehyde

To a 5 L 3-neck flask equipped with overhead mechanical stirrer, thermometer, condenser, and nitrogen line was added chrysene (Eastman Kodak Co., Rochester, N.Y. 14650, 100 g, 0.438 mole) and o-dichlorobenzene (2500 mL). The liquid was warmed until all the large chunks of solid dissolved (80°) and then cooled quickly to give finely divided crystals. After further cooling with a salt-ice bath to 5°, $SnCl_4$ (Aldrich Chemical Co., Milwaukee, Wis.. 53201, 98%, 228.2 g, 0.876 mole, 102.4 mL), was added in one portion. No temperature change occurred. Keeping the pot temperature below 5° α,α-dichloromethyl methyl ether (Aldrich, 70.48 g, 0.613 mole, 55.45 mL) was added dropwise over 1 hour. The resulting suspension was warmed slowly to 40° over 4 hours and further stirred for 16 hours. Considerable HCl gas evolution occurred during the warming and the early part of the reaction at 40°. The reaction was then cooled to 10° and hydrolysed by careful addition of 1 L of cold $H_2O$. After 4 hours the layers were separated and the organic layer filtered, dried with anhydrous $Na_2SO_4$ (Mallinckrodt Co., St. Louis, Mo., 100 g) and filtered again. The clear yellow solution was split into 2 portions and passed through 500 g plugs of "flash chromatography" silica gel (E. Merk, silica gel 60, 230—400 mesh) using toluene as the eluting solvent with 500 mL fractions. This separated unreacted chrysene (3 g) from the aldehyde and a more polar product. Fractions containing the aldehyde were combined and the toluene removed. Crystals formed during this process and were removed periodically by filtration. After drying in a vacuum oven (at 60°) final yield of 6-chrysenecarbaldehyde was 89.46 g (79.7%) mp = 167—196°.

Example B

10-Methylthio-9-anthracenecarbaldehyde

The procedure of V. Rogovik et al., *Zh. Org. Khim. 3,* 1315 (1969) was modified in the following way: A 2 L 3-neck flask fitted with stirring bar, condenser, additional funnel, thermometer, $N_2$ inlet, and bubbler was charged with 10-chloro-9-anthracenecarbaldehyde (Aldrich, 28.0 g, 0.116 mol), and DMF (Aldrich, 1 L). The solid dissolved when the reaction mixture was warmed to 60°. The addition funnel was filled with a solution of $Na_2S$ (Mallinckrodt, 28 g, 0.116 mol) in 30 mL of $H_2O$. This solution was added rapidly to the flask causing a considerable amount of spattering as the purple thiolate formed. The reaction mixture was stirred at 65° for 45 minutes, then cooled to 30° (ice bath). $CH_3I$ (Aldrich, 27.36 g, 0.193 mol) was then added to the flask dropwise over 5 minutes. The colour of the solution changed from deep purple to yellow after 3 hr. After 15 minutes, 1 L of $H_2O$ was added to the reaction mixture. The yellow solid that formed was collected by filtration, dissolved in hot toluene, (500 mL) dried ($MgSO_4$), and filtered through Celite (Trade Mark). Most of the volume of toluene was removed and the resultant oil swirled with hexane (200 mL) to give a bright yellow solid. The material was dried at 50° affording 25.04 g (86%) of 10-methylthio-9-anthracenecarbaldehyde mp 98.5—99°, (C, H, S).

Example C

10-(2-Chloroethyl)-9-anthracenecarbaldehyde

Using the Vilsmeier procedure (L. F. Fieser, *Org. Syn. Coll. Vol III,* 98 (1955)), 9-vinylanthracene (Aldrich) gave 10-(2-chloroethyl)-9-anthracenecarbaldehyde mp 158—159°, ($PhCH_3/CH_3OH$), (C, H, Cl).

Example D

A. 1,10-Dichloro-9-anthracenecarbaldehyde and 4,10-Dichloro-9-anthracene-carbaldehyde

Using the procedure of V. I. Rogovik et al., *Zh. Org. Khim 3,* 1315 (1967), 1-chloroanthraquinone (Aldrich) gave a mixture of 1,10- and 4,10-dichloro-9-anthracenecarbaldehydes. These compounds were separated by preparative HPLC using toluene as the eluting solvent to give 3.05 g (14%) of 1,10-dichloro-9-anthracenecarbaldehyde mp 180.5—183°, ($R_f$ = 0.64, $SiO_2$, $PhCH_3$), (C, H, Cl), and 0.59 g (3%) of 4,10-dichloro-9-anthracenecarbaldehyde mp 167—170°, ($R_f$ = 0.57, $SiO_2$, $PhCH_3$), (C, H, Cl).

Example E

A. 10-Bromo-9-anthracenecarbaldehyde

This material was made from 9,10-dibromoanthracene (Eastman 20 g, 60 mmol) modifying the procedure of R. Kuhn and H. Fischer, *Chem. Ber. 94,* 3060 (1961). In this procedure, the reaction mixture was cooled to −78° before the nBuLi was added. The resulting mixture was warmed to RT over 1 hr and then refluxed until the crystalline starting material disappeared. The mixture was then cooled to −78° again before the DMF was addded (in one portion). The flask was warmed to RT and then quenched with 1M HBr (200 mL). The two-phase system was then extracted with $CH_2Cl_2$ (3 × 500 mL). The extracts were combined, dried ($MgSO_4$), filtered, and the solvent removed to give the crude material. This was purified by preparative HPLC using toluene as the eluting solvent. After the solvent was removed 13.06 g (76%) of 10-bromo-9-anthracenecarbaldehyde mp 215—216.5°, (lit, mp 218°, P. Kuhn a nd H. Fischer, *Chem. Ber. 94,* 3060 (1961)), (C, H, Br) was obtained.

Example F

4,5-Dichloro-9-anthracenecarbaldehyde

1,8-Dichloroanthracene prepared by the method of H. O. House et al. (*J. Org. Chem. 38,* 1167 (1973)), was formylated by the method outlined in A (except that $CH_2Cl_2$ was used as the reaction solvent) to give 4,5-dichloro-9-anthracenecarbaldehyde mp 218—220°, ($PhCH_3/CH_3OH$), (C, H, Cl), (lit. 224—226°, E. L. Stogryn, *J. Med. Chem. 17,* 563 (1974)).

Example G

Formylation of Fluoranthene

Fluoranthene (Aldrich, 100 g, 0.49 mol) was formylated according to the procedure outlined in A (except that $CH_2Cl_2$ was used as the reaction solvent). The crude material was passed through a 1000 g plug of $SiO_2$ using toluene as the eluting solvent (3 L). The fractions containing the mixtures of aldehydes were combined and the solvent removed giving 115 g of crude yellow oil. This material was dissolved in 500 mL of $CH_2Cl_2$ and diluted to 1 L with hexane. A yellow precipitate formed and this was isolated by filtration. The solid (which is 3-fluoroanthene-carbaldehyde) was crystallized from $CH_2Cl_2$/hexane and dried at 50° to give 45.7 g of pure material. The filtrate was added to the remaining impure mixture and the solvent removed. The remainder of the material was chromatographed on a 1000 g plug of $SiO_2$ using $PhCH_3$ as eluting solvent. From this mixture, three aldehydes (including more of the 3-isomer) were obtained. The total amounts isolated, identity, and TLC behaviour ($SiO_2/PhCH_3$) of these aldehydes are shown below.

I. *3-Fluoranthenecarbaldehyde* 68.73 g (61%) mp 103—104.5°, ($R_f$ = 0.27, (C, H), (lit. mp 98—99°, N. Campbell and N. H. Wilson, *Chem. and Ind.,* 1114, (1970).

II. *7-FLuoranthenecarbaldehyde* 2.10 g (2%) mp 139—141°, (C, H), $R_f$ = 0.38.

III. *8-Fluoranthenecarbaldehyde* 24.8 g (22%) mp 91.5—93°, (C, H), ($R_f$ = 0.19).

Example H

4-Chloro-9-anthracenecarbaldehyde

1-Chloroanthracene prepared from 1-chloroanthraquinone (Aldrich) by the method of H. O. House et. al. (*J. Org. Chem. 38,* 1167, (1973)) was formylated by the procedure outlined in A (except that $CH_2Cl_2$ was used as the reaction solvent to give 4-chloro-9-anthracenecarbaldehyde mp 129—131°, ($PhCH_3/CH_3OH$), C, H, Cl).

Example I

10-Methylsulfinyl-9-anthracenecarbaldehyde

A 1 L round bottom flask fitted with addition funnel and stirring bar was charged with 10-methylthio-9-anthracenecarbaldehyde (example B, 12.0 g, 48 mmol) and 450 mL of $CH_2Cl_2$. The resulting solution was cooled to 5° with an ice bath. A solution of MCPBA (Aldrich (85%), 9.64 g, 48 mmol) in 350 mL of $CH_2Cl_2$ was then added dropwise to the flask over 1 hr. The reaction mixture was allowed to warm to RT over 1 hr and then was washed with 5% $NaHCO_3$ solution (2 × 500 mL), dried ($Na_2SO4$), filtered, concentrated to 500 mL, and passed through $SiO_2$ (250 g) using toluene (5 L) as the eluting solvent. The desired material was then eluted from the $SiO_2$ using EtOAc (2 L) as the eluting solvent. The solvent volume was reduced to 100 mL and then filtered to 700 mL with hexane. The resulting yellow solid was filtered and dried at 50° to give 11.98 g (94%) of 10-methylsulfinyl-9-anthracenecarbaldehyde mp 182—184°, (C, H, S).

Example J

2-Triphenylenecarbaldehyde

Using the formylation procedure described in A (except that the reaction tèmperature was 85°), triphenylene (Aldrich) gave 2-triphenylenecarbaldehyde mp 160—161.5°, ($CH_2Cl_2/CH_3OH$), (C, H).

Example K

10-Methoxy-9-anthracenecarbaldehyde

A 2 L round bottom flask fitted with distilling head, thermometer, and condenser was charged with 15-crown-5 (Aldrich, 25.89 g, 0.118 mol), $NaOCH_3$ (Aldrich, 7.62 g, 0.141 mol), and $CH_3OH$ (50 mL). After 5 minutes 10-chloro-9-anthracenecarbaldehyde (Aldrich 28.4 g, 0.118 mol) and 900 mL of dry toluene were added to the clear colourless solution. The solvent was distilled off until the head temperature reached 108° (300 mL). Additional dry toluene was added to give a total of 1 L volume. The reaction mixture was refluxed for 4 hr, cooled and poured onto a large plug of $SiO_2$ (1000 g) in a sintered glass funnel. The crude product was chromatographed using toluene as eluent (5 L). The fractions (250 mL) containing the product were combined (≃ 3 L) and the solvent volume reduced to 500 mL. The shiny golden crystals which formed were filtered to give, after drying at 50°, 15.6 g of material. The volume of the filtrate was reduced to 200 mL and more material fell out of solution and this was filtered and dried to give 6.1 g of additional material. The two crops were combined to give 22.51 g (81%) of 10-methoxy-9-anthracenecarbaldehyde which was used without further purification. Recrystallization gave analytically pure material mp 164.5—166.5°, ($PhCH_3$), (C, H), (lit. mp 165°, J. B. Conant and M. Bramann, *J. Amer. Chem. Soc. 50,* 2305 (1928)).

Example L

10-Formyl-9-anthracenecarbonitrile

A 25 mL 2-neck round bottom flask fitted with thermometer, condenser, $N_2$ inlet and bubbler, and stirring bar was charged with 10-chloro-9-anthraldehyde (Aldrich, 5 g, 21 mmol), CuCN (Fisher Scientific Company, 711 Forges Ave., Pittsburgh, PA, 15219, 2.14 g, 24 mmol), N-methyl-pyrrolidinone (100 mL), DMF (15 mL), and bis (triphenylphosphine) palladium dichloride (Fluka, 0.08 g, 01. mmol). The mixture was warmed to 170° and stirred 15 hr under $N_2$. After 1.5 hr, the mixture became homogenous. The reaction was cooled to 70° and poured into a solution composed of 16 g of $FeCl_3.6H_2O$, (Mallinckrodt), 70 mL of 1.0 M HCl and 400 mL $H_2O$. The resulting mixture was stirred at 60—70° for 1 hr, filtered and a crude orange solid isolated. This material was dissolved in 1 L of hot toluene and passed through a small plug (100 g) of $SiO_2$. The filtrate was then concentrated to 75 mL and diluted with hexane (200 mL). The orange solid which formed was collected by filtration and dried to give 3.17 g (68%) of 10-formyl-9-anthracenecarbonitrile mp 270—275°, (C, H, N).

Example M

9,10-Dihydro-9,10-dioxo-1-anthracenecarboxylic acid

Benzanthrone (Aldrich, Technical grade) was purified by chromatography on a plug of $SiO_2$ with $PhCH_3$ as eluent (83% recovery). mp 172—172.5° (lit. mp. 170—171°, O. Bally and R. Scholl, *Ber. 44,* 1656 (1911)).

The purified benzanhrone (63.7 g, 0.277 mol) was dissolved in 15 mL of glacial HOAc at 90° and stirred with a mechanical stirrer. After cooling to 80° solid $CrO_3$ (Mallinckrodt 200 g, 2 mol) was added in ≃5 g portions over about 4 hr. The exothermic reaction maintained the mixture at ≃80° during this time and $CO_2$ was evolved. After $CO_2$ evolution ceased and the reaction temperature fell, the heating mantle was reapplied and the reaction stirred overnight. $H_2O$ (1.5 L) was then added to the dark-green solution. The reaction was then filtered to give a deep brown solid which was washed with $Ch_3OH$ (200 mL) until the washings were colourless. The resulting solid was dissolved 2 L of hot methoxyethanol and filtered through Celite (Trade Mark) to remove a black solid residue. The volume of the solution was reduced to ≃75 mL (some solid formed) and diluted with 100 mL $CH_3OH$ to give the product. This material was filtered to give 32.0 g (46%) of golden brown 9,10-dihydro-9,10-dioxo-1-anthracenecarboxylic acid mp 287—289°, (C, H), (lit. mp 293—294°, Chemistry of Carbon Compounds IIIb, edited by E. H. Rodd, 1419 (1956), Elsevier, New York.

1-Anthracenecarboxylic acid

To a 5 L 3-neck flask fitted with condenser, thermometer, and overhead stirrer was added 9,10-dihydro-9,10-dioxo-1-anthracenecarboxylic acid (90 g, 0.357 mol), zinc dust (Mallinckrodt, 250 g, 3.82 mol), $CuSO_4.5H_2O$ (Mallinckrodt, 5 g), and 28% $NH_4OH$ (Mallinckrodt, 2500 mL). The mixture was heated slowly until a dark-red solution occurred as the temperature reached 85°. After 3.5 hr the colour of the solution faded to yellow. The reaction was heated an additional 1 hr, and then cooled and the excess zinc filtered. The filter cake was washed with more $NH_4OH$ (100 mL) and then discarded. The filtrate was carefully acidified to pH 1 with conc. HCl added in portions over 1 hr affording a light-green precipitate which was separated by filtration. The solid was washed with $H_2O$ (200 mL) and then recrystallized once from methoxy- ethanol/$H_2O$ (with a small amount of HCl) filtered, and dried at 75° to give 65 g (82%) of 1-anthracenecarboxylic acid mp 249—250°, (C, H), (lit. mp 245°, Chemistry of Carbon Compounds IIIb, edited by E. H. Rodd, 1373 (1956), Elsevier, New York).

(1-Anthryl)methanol

To a 500 mL 2-neck flask equipped with condenser, addition funnel with $N_2$ inlet, and stirring bar was added 1-anthracenecarboxylic acid (6.88 g, 31 mmol) and dry THF (250 mL). To the addition funnel, was added a 1M solution of $BH_3$ in THF (Aldrich, 50 mL, 50 mmol) via a cannula. The $BH_3$ solution was added over 1 hr and the solution stirred overnight at RT. $CH_3OH$ was then added until $H_2$ evolution ceased. $H_2O$ (5

mL) and then 1N HCl (5 mL) was added to the flask. The solvents were removed and then toluene (100 ml) added to the flask. The toluene was then also removed. The resulting solid was recrystallized from EtOAc/hexane to give 4.3 g (67%) of (1-anthryl)-methanol mp 124—125°, (C, H), lit 124—125°, S. Akiyama et al., *Bull. Chem. Soc. Jap.* 35 (1962)).

1-Anthracenecarbaldehyde

To a 2 L round bottom flask equipped with condenser and magnetic stirring bar was added (1-anthryl)methanol (21.0 g, 0.10 mol, $CH_2Cl_2$ (1200 mL) and pyridinium chlorochromate (PCC) (Aldrich, 32.33 g, 0.15 mol). The mixture was then refluxed for 5 hr. The reaction was cooled and then filtered through a 400 g plug of silica gel using toluene as eluting solvent. The first 1 L of solution was collected and concentrated to give 16 g of crude product. This material was purified by preparative HPLC using $PhCH_3$ as eluting solvent. The solvent was removed and the pure material recrystallized from $PhCH_3$/hexane to give 14.0 g (67%) of 1-anthracenecarbaldehyde mp 130—131.5°, (C, H), (lit. mp 126.5—127.5°, P. H. Gore *J. Chem. Soc.* 1616 (1959)).

Example N

(10-Bromo-1-anthryl)methanol

10-Bromo-1-anthracenecarboxylic acid, made from 1-anthracenecarboxylic acid (example M) by the procedure of E. Barnett, J. W. Cook, and H. H. Grainger, *Ber. 57* B, 1775 (1924), was reduced with $BH_3$ in THF by the procedure outlined in 18C to give (10-bromo-1-anthryl)methanol mp 125—127°, (EtOAc/hexane), (C, H, Br).

10-Bromo-1-anthracenecarbaldehyde

Using the procedure outlined in example M oxidation of (10-bromo-1-anthryl)methanol with PCC gave 10-bromo-1-anthracenecarbaldehyde mp 134.5—135.5°, ($PhCH_3$/hexane), (C, H, Br).

Example O

2-Chloro-9-anthracenecarbaldehyde and 3-chloro-9-anthracenecarbaldehyde

2-Chloroanthracene prepared from 2-chloroanthraquinone (Aldrich) by the method of H. O. House et al. (*J. Org. Chem. 38,* 1167 (1973)) was formylated by the procedure outlined in A (except that $CH_2Cl_2$ was used as the reaction solvent) to give a (4:1) mixture of 2-chloro and 3-chloro-9-anthracenecarbaldehydes (87%). Trituration of the material with $CH_3OH$ gave preferential crystallization of 2-chloro-9-anthracenecarbaldehyde which after further crystallization ($PhCH_3$/hexane) gave the pure 2-chloro isomer mp 149—150° (C, H, Cl) (lit. 148—150°, British Patent 1,149,557). The filtrate ($R_f = 0.48$, $SiO_2$, $PhCH_3$) from the $CH_3OH$ trituration was further purified by preparative HPLC to give pure 3-chloro-9-anthraldehyde mp. 122—123.5°, ($PhCH_3$/hexane), (C, H, Cl), ($Rf = 0.48$, $SiO_2$, $PhCH_3$).

Example P

10-Ethylthio-9-anthracenecarbaldehyde

Using the procedure described in Example B 10-chloro-9-anthracenecarbaldehyde (Aldrich) and ethyl iodide (Fisher) gave an oil which solidified to give 10-ethylthio-9-anthracenecarbaldehyde mp 74—75.5° (C, H, S).

Example Q

10-((2-Hydroxyethyl)thio)-9-anthracenecarbaldehyde

Using the procedure described in example B (except that the alkylation reaction was run for 1 hr at 65°), 10-chloro-9-anthracenecarbaldehyde (Aldrich), and 2-iodoethanol (Aldrich) gave 10-((2-hydroxyethyl)thio-9-anthracenecarbaldehyde mp 103—104°, ($PhCH_3$/hexane), (C, H, S).

Example R

2,10-Dichloroanthracenecarbaldehyde and 3,10-dichloro-9-anthracene carbaldehyde

Using the procedure of V. I. Rogovik et al. (*Zh. Org. Khim. 3,* 1315 (1967)) 2-chloroanthraquinone (Aldrich) gave a mixture ($\simeq$1:1) of 2,10- and 3,10-dichloroanthracenecarbaldehydes (68%). A portion of the mixture was separated by preparative HPLC using the shave/recycle technique to give 2,10-dichloro-9-anthracenecarbaldehyde mp 175.5—176.5°, ($PhCH_3$), (C, H, Cl), and 3,10-dichloro-9-anthracenecarbaldehyde mp 173.5—175°, ($PhCH_3$), (C, H, Cl). The remainder of the material was used as a mixture.

Example S

10-Ethoxy-9-anthracenecarbaldehyde

Using the procedure outlined in example K, except that NaOEt (Aldrich) EtOH was used instead of $NaOCH_3/CH_3OH$, 10-chloro-9-anthraldehyde (Aldrich) gave 10-ethoxy-9-anthracenecarbaldehyde mp 88—90°, ($CH_2Cl_2$/hexane (C, H).

## Example T

10-(2-hydroxyethyloxy)-9-anthracenecarbaldehyde

A 3 L 2-neck flask fitted with thermometer, condenser, stirring bar, $N_2$ line and bubbler was charged with KO*t*Bu (MCB Manufacturing Chemists. Inc., 2909 Highland Ave., Cincinnati, OH, 45212, 25 g, 0.22 mol), ethyleneglycol (1500 ml) and 10-chloro-9-anthraldehyde (Aldrich, 50 g, 0.207 mol). The mixture was stirred at 100° for 1.5 h. An additional 5 g (45 mmol) of KO*t*Bu was added and the stirring continued for an additional 0.5 h. The reaction mixture was cooled and poured into 1500 mL of cold $H_2O$, stirred for 10 minutes before the precipitate was collected by filtration. The yellow solid was dissolved in 1 L of $CH_2Cl_2$ and passed through a 100 g plug of $SiO_2$ using $CH_2Cl_2$ (9.L). The $CH_2Cl_2$ was discarded and the desired material eluted with EtOAC (12 L). The appropriate fractions were combined and the solvent removed to give after drying 50° 10-(2-hydroxyethyloxy)-9-anthracenecarbaldehyde 28.82 g (53%), mp 142—144°, ($CH_2Cl_2$/hexane), (C, H).

## Example U

10-Methylsulfonyl-9-anthracenecarbaldehyde

10-Methylthio-9-anthracenecarbaldehyde (4.50 g, 17.83 mmol) was dissolved in $CH_2Cl_2$ (100 ml) and cooled to 0° in an ice bath. To the magnetically stirred solution was added dropwise over 15 minutes a solution of m-chloroperbenzoic acid (Aldrich, 85% technical grade, 7.08 g 35.76 mmol) in 250 ml of $CH_2Cl_2$. The ice bath was removed and the clear solution stirred for 2 h. The solution was then washed sequentially with 10% $Na_2S_2O_3$ solution (500 ml) and satd. $Na_2CO_3$ solution (2 × 100 ml). The solvent was removed and the crude material passed through a small plug of silica gel (200 ml) in a sintered glass funnel using $CH_2Cl_2$ as the eluting solvent (500 ml). The solvent was removed to give the crude product which was recrystallized from $CH_2Cl_2$/EtOH to give 10-methylsulfonyl-9-anthracenecarbaldehyde mp 216—217° (C, H, S).

## Example V

10-(2-Methoxyethoxy)-9-anthracenecarbaldehyde

KO*t*Bu (MCB Manufacturing Chemists, Inc. 18.2 g, 0.162 mole) in methoxyethanol (1000 ml) was treated with 10-chloro-9-orthraldehyde (Aldrich, 25 g, 0.104 mole) and heated at reflux for 2 h. The cooled reaction mixture was diluted with $H_2O$ (5 L) and the resulting oil stirred for 2 h until solidification occurred. The filtered solid was chromatographed on a plug of $SiO_2$ (500 g) using $CH_2Cl_2$ as the eluting solvent to afford 26.9 g (92%) of 10-(2-methoxyethoxy)-8-anthracenecarboxaldehyde mp 87—88°, (C, N), ($CH_2Cl_2$/hexane), (Rf = 0.16, $SiO_2$, $CH_2Cl_2$).

## Example W

10-Morpholino-9-anthracenecarbaldehyde

10-Chloro-9-anthracene carboxaldehyde (Aldrich, 25 g, 0.104 mole) in morpholine (MCB, practical, 500 ml) was heated at 55° under $N_2$ for 17 h. The reaction mixture was poured into $H_2O$ (2 L). The filtered precipitate was chromatographed on a plug of $SiO_2$ (1 kg) using toluene (4 L) as the initial eluting solvent to removed starting material and byproducts. The orange product band was then eluted with $CH_2Cl_2$ (2 L) to yield 10.58 g (35%) of 10-morpholino-9-anthracenecarboxaldehyde mp 182—184° softens 175°), (C, H, N), (Rf = 0.16, $SiO_2$, $CH_2Cl_2$).

## Example X

12-chloro-6-chrysenecarbaldehyde

6-Chlorochrysene (Cambridge Chemical. 70 g 0.266 mole) was formylated according to the procedure outlined in example 1A, except that $CH_2Cl_2$ (2500 ml) was used as the reaction solvent. Chromatography on a plug of $SiO_2$ (1 kg) using EtOAc as the eluting solvent afforded 19.1 g (25%) of 12-chloro-6-chrysenecarbaldehyde mp 255—257°, (EtOAc), (Rf = 0.42, $SiO_2$, toluene).

## Example Y

10-(Imidazol-1-yl)-9-anthracencarbaldehyde

A solution of 10-chloro-9-anthraldehyde (Aldrich, 15 g, 0.062 mole), imidazole (Aldrich, 10.2 g 0.15 mole), and DMF (300 ml) at 55° was treated with KOtBu (MCB, 7.9 g 0.07 mole) and stirred for 30 minutes. The reaction mixture was poured into 0.1 M NAOH (1500 ml). The filtered precipitate was chromatographed on a plug of $SiO_2$ (500 g) using $CH_2Cl_2$ (3 L) as the initial eluting solvent to remove starting material and byproducts. The yellow product band was then eluted with EtOAc (2 L) to yield 12.29 g (73%) of 10-(imidazol-1-yl)-9-anthracenecarbaldehyde mp 194—196°, (C, H, N), EtOAc), (Rf = 0.38, $SiO_2$, EtOAc).

## Example Z

2-Ethylanthracene

To a 5 L 3-neck flask fitted with condenser, thermometer, and overhead stirrer was added 2-ethylanthraquinone (Aldrich, 120 g, 0.51 mol), Zn dust (Mallinckrodt, 300 g, 4.59 mol), $CaSO_4.5\ H_2O$ (Mallinckrodt, 3.0 g), and 28% $NH_4OH$ (Mallinckrodt, 2800 mL). The temperature was increased until the initial dark red colour had faded (about 6 h). The reaction mixture was then filtered. The filtrate was extracted with EtOAc, and the zinc solid also extracted with EtOAc. The EtOAc solutions were combined

and the solvent removed. The residue was refluxed with a mixture of *con* HCl (10 mL) in p-PrOH (1200 mL) for 2 h. Upon cooling, a solid precipitated which was filtered, washed with abs. EtOH (100 mL) and dried to give 40 g (38%) of 2-ethylanthracene mp, C, H.

2- and 3-Ethylanthracene-9-carbaldehyde

2-Ethylanthracene (40 g, 0.194 mol) was formylated according to the procedure outlined in example A, except that $CH_2Cl_2$ (500 mL) was used as the reaction solvent. Chromatography over a plug of $SiO_2$ with $PhCH_3$ as the eluting solvent gave 43.68 g (96%) of a mixture of 2- and 3-ethyl-anthracene-9-carbaldehyde.

Example AA

3,5-Diphenyl-7a(7H)-ethoxymethyl-1H,3H,5H-oxazolo(3,4-c)oxazole

A mechanically stirred 60% dispersion of NaH in mineral oil (Alfa-Ventron, 34.0 g, 0.85 mol) was washed with dry hexane to remove the oil and suspended in dry DMF (300 mL). To the mixture was added a solution of 3,5-diphenyl-1H,3H,5H-oxazolo(3,4-c)oxazole-7a(7H)-methanol (208.2 g, 0.7 mol, prepared by the method of J. Pierce et al JACS *73* 2595 (1951)) in dry DMF (300 mL) keeping the reaction mixture between 30—35°. The salt suspension was stirred at RT for 60 min, diluted with dry DMF (200 mL) to facilitate stirring, cooled, then treated with ethyl iodide (Aldrich, excess) at such a rate that the reaction temperature was between 20—35°. The mixture was stirred at RT for 2 h, then cautiously treated with absolute EtOH (30 mL). The resulting mixture was diluted with $Et_2O$ (2.5 L) and the resulting solids removed by filtration. The solvent was then removed using a rotary evaporator to give 229.5 g of a yellow oil containing both starting material and desired product. A solution of the oil in chloroform was mixed with $SiO_2$ (200 g) and the solvent removed. The solid was then added to a column of $SiO_2$ (800 g). Elution with the EtOAc/hexane (1:3.5) gave 139.7 g (61.3%) of 3,5-diphenyl-7a(7H)-ethoxymethyl-1H,3H,5H-oxazolo(3,4-c)oxazole. An analytical sample was obtained by recrystallization from hexane, mp of 83.5—85°, (C, H, N). The bulk of the material was used without further purification.

2-Amino-2-ethoxymethoxy-1,3-propanediol hydrochloride $1/4H_2O$

3,5-Diphenyl-7a(7H)-ethoxymethyl-1H,3H,5H-oxazolo(3,4-c)oxazole (136 g, 0.42 mol) was dissolved in 6 N HCl (400 mL) and the resulting solution stirred 1.5 h at RT. After extraction with $Et_2O$ (2 × 200 mL) to remove benzaldehyde, the aqueous solution was concentrated on a rotary evaporator to give a colourless oil. This was cooled in an ice bath to facilitate crystallization. The solid which formed was slurried with cold $CH_3CN$, filtered, then washed with $Et_2O$ and dried in a vacuum oven at RT to give 71 g (89%) of 2-amino-2-ethoxymethyl-1,3-propanediol hydrochloride 1/4 $H_2O$ mp 78—79°, (C, H, Cl, N).

Example AB

4-Aza-3-hydroxymethyl-3-methyl-1-oxaspiro(4,5)decane

A solution of 2-amino-2-methyl-1,3-propanediol (Aldrich, 303.4 g, 3.0 mol), cyclohexanone (Fisher, 294.5 g, 3.0 mol) and $PhCH_3$ (400 mL) was refluxed for approximately 2 h with azeotropic removal of $H_2O$. The material which crystallized from the $PhCH_3$ on cooling was recrystallized twice from hexane to give 444.4 g of 4-aza-3-hydroxymethyl-3-methyl-1-oxaspiro(4.5)decane (80%) mp 52—54°, (C, H, N).

4-Aza-3-methoxymethyl-3-methyl-1-oxaspiro(4.5)decane

A mechanically stirred 60% dispersion of NaH in mineral oil (Alfa-Ventron, 75 g, 1.9 mol) was washed with dry hexane to remove the oil and suspended in dry DMF (200 mL). To the mixture was added a solution of 4-aza-3-hydroxymethyl-3-methyl-1-oxaspiro(4.5)decane (27.8 g, 1.5 mol) in dry DMF (200 mL) keeping the reaction mixture temperature between 30—35°. Small amounts of DMF were added as necessary to facilitate stirring. The mixture was stirred at RT for 1.5 h, then cooled and treated with methyl iodide (Fisher, 234.2 g, 102.7 mL, 1.65 mol) keeping the reaction temperature between 20—30°. The mixture was stirred 2 h at RT and slowly treated with absolute EtOH (40 mL), then diluted with dry $Et_2O$ (3 L). The reaction mixture was filtered and the solvent removed by rotary evaporation. The residue was then fractionally distilled to give 209.7 g (73.3%) of 4-aza-3-methoxymethyl-3-methyl-1-oxaspiro(4.5)decane as a colourless liquid bp 114°/14 mm, (C, H, N).

2-Amino-3-methoxy-2-methyl-1-propanol

A solution of 4-aza-3-methoxymethyl-3-methyl-1-oxaspiro(4.5)decane (299 g, 1.5 mol) and 6 N HCl (500 mL) was refluxed for 60 min. On cooling, two layers formed, the upper one containing cyclohexanone was removed by extraction with $Et_2O$ (2 × 400 mL). The lower aqueous layer was concentrated on a rotary evaporator to give a syrup which then was treated with excess 50% NaOH. The resulting slurry was extracted with $Et_2O/CH_2Cl_2$ (2:1, 4 × 500 mL), then with $CH_2Cl_2$ (500 mL). The solvent was removed by rotary evaporation to give 198 g of pale oil. Fractional distillation of this oil gave 166 g (93%) of 2-amino-3-methoxymethyl-1-propanol as a colourless oil, bp 94°C/17 mm, (C, H, N).

Example AC

1α,2α,3α-2-Amino-1,3-cyclohexanediol acetate

This compound was prepared by the method of F. Lichtenthaler (Ber, 96, 851 (1963)), mp 175—177°, (C,

H, N), (lit 175—177°, F. Lichtenthaler, Ber. 96, 851 (1963)).

Example AD

2-Isopropyl-2-nitro-1,3-propanediol

A solution of 2-methyl-1-nitropropane (38.7 g, 0.375 mol prepared by the procedure of N. Kornblum, B. Tunbe, and H. Ungnade, J. Am. Chem, Soc., 1954, 76, 3029) and $NEt_3$ (Eastman 3.79 g, 0.0375 mol) in $CH_3OH$ (50 mL) was added dropwise to 37% aqueous formaldehyde solution (Mallinckrodt 76.2 g, 0.938 mol) at a rate such that the reaction mixture temperature did not exceed 30°. After 72 h, the solution was concentrated under vacuum and the residue was dissolved in $H_2O$ (250 mL). The solution was continuously extracted for 1 h with $CH_2Cl_2$ (1 L). The $CH_2Cl_2$ solution was dried ($MgSO_4$), filtered, and concentrated to give 53.3 g of 2-isopropyl-2-nitro-1,3-propanediol a waxy, white solid (87%); mp 67—72°C (lit. mp 87—88°, B. M. Vanderbilt and H. B. Hass, Ind. Eng. Chem. *32,* 34 (1940). In our hands this procedure failed to give the desired compound).

2-Amino-2-isopropyl-1,3-propanediol acetate

Using the procedure in example AM, 2-isopropyl-2-nitro-1,3-propanediol gave a 98% yield of 2-amino-2-isopropyl-1,3-propanediol acetate mp 155—155.5°. (H. S. Broadbent et al. J. Hetercyclic Chem., *13,* 337 (1975) report the synthesis of this compound as the free base (mp 70—72°C)).

Example AE

Ethyl N-benzylidene-L-alaninate

Ethyl N-benzylidene-L-alaninate was prepared according to the general procedure of G. Stork et al., J. Org. Chem. *41,* 249 (1976), bp 98—100°/0.4 mm (lit. 100°/0.3 mm, A. Calcayai et al., Synthesis 445 (1981)).

2-(2-Iodoethoxy)tetrahydro-2H-pyran

Freshly distilled dihydropyran (Aldrich, 59.0 g, 0.7 mol) was added dropwise to a cooled solution of iodoethanol (Aldrich, 98 g, 0.57 mol) in $Et_2O$ (1 L) containing 0.1 g of p-toluenesulfonic acid (Eastman). The solution was then stirred for 1 h at 5°. Solid $K_2CO_3$ (Mallinckrodt, 5 g) was then added to the reaction mixture and the resulting suspension stirred an additional 1 h at RT. The reaction was then filtered and the remaining solid washed with $Et_2O$ (1 L). The organic solutions were combined and concentrated on a rotary evaporator (in a flask washed with 1% $NEt_3$ in $H_2O$). The crude 2-(2-iodoethoxy)-tetrahydro-2H-pyran (100 g, 68.9%) was used without further purification.

Ethyl 2-benzylideneamino-2-methyl-4-((tetrahydro-2H-pyran-2-yl)oxy)butyrate

A solution of lithium diisopropylamide was prepared by dropwise addition of n-BuLi (Aldrich 1.6 M in hexane, 228 mL, 0.365 mol) to a solution of diisopropylene (Aldrich, 51.6 g, 0.51 mol) in a mixture of dry THF (700 mL) and dry HMPA (Aldrich, 40 mL) kept at 30—40°. The solution was then cooled to −70° and a solution of ethyl N-benzylidene-L-alaninate (74.9 g 0.365 mol) was added dropwise to the solution allowing the reaction mixture to warm to −20° for several min. The resulting red solution was then cooled to −70°.

2-(2-Iodoethoxy)tetrahydro-2H-pyran (98.1 g, 0.383 mol) was then added to the solution at such a rate that the temperature in the reaction mixture did not rise above −65°. The solution was allowed to warm slowly to RT and stirred for 14 h. The volume of the solution was reduced to 300 mL by a stream of dry $N_2$ during the last few hours to facilitate the final workup. The reaction was quenched with sat. NaCl (800 mL) and diluted with $Et_2O$ (800 mL). The $Et_2O$ was removed and the aqueous layer extracted with hexane (500 mL). The $Et_2O$ and hexane layers were combined and dried ($Na_2SO_4$). The solution was filtered and the solvent removed to give 124 g of crude red oil. Bulb to bulb distillation (in 1% aq. $NEt_3$ washed glassware) (210° bath temperature/0.3 mm) gave 95 g of ethyl 2-benzylideneamino-2-methyl-4-((tetrahydro-2H-pyran-2-yl)oxy)butyrate which was homogeneous by vpc and gave acceptable NMR and mass spectra. It was stored under $N_2$ in the refrigerator and was used without further purification.

2-benzylamino-2-methyl-4-((tetrahydropyran-2-yl)oxy)butanol

A solution of ethyl 2-benzylideneamino-2-methyl-4-((tetrahydro-2H-pyran-2-yl)oxy)butyrate (100.0 g, 0.3 mol) in THF (100 mL) was added slowly to a suspension of lithium aluminum hydride (Alfa-Ventron, 22.77 g, 0.6 mol) rapidly stirred in dry THF (1 L) at such a rate to maintain a gentle reflux. After the addition was complete the mixture was refluxed for 4 h. The reaction mixture was cooled and treated successively with $H_2O$ (23 mL), 15N NaOH (23 mL) and $H_2O$ (45 mL). The solid was removed by filtration and washed with THF (200 mL). The organic layers were combined and concentrated by rotary evaporation to give 2-benzylamino-2-methyl-4-((tetrahydropyran-2-yl)oxy)butanol (81.1 g, 92.0%) as a thick oil which was used without further purification.

2-Benzylamino-2-methyl-1,4-butanediol

The crude-2-benzylamino-2-methyl-4-((tetrahydropyran-2-yl)oxy)butanol (80.1 g, 0.273 mol) was dissolved in 3N HCl (128 mL). After 5 min the mixture was washed with $Et_2O$ (200 mL). The aqueous solution was concentrated by rotary evaporation to give a thick oil which was cooled and basified with excess 50% NaOH. The oily amine which formed was extracted with $Et_2O$ (3 × 200 mL). The $Et_2O$ extracts

were combined and concentrated to give 63.6 g of a thick oil. Distillation gave 49.8 g (94%) of 2-benzylamino-2-methyl-1,4-butanediol as a pale yellow oil (bp 168—170°/0.35 mm) (C, H, N).

2-Amino-2-methyl-1,4-butanediol hydrochloride

2-Benzylamino-2-methyl-1,4-butanediol (31.08 g, 0.149 mol) was dissolved in 95% EtOH (240 mL) containing *Con* HCl (21 mL, 0.25 mol) and 5% Pd/C (10.0 g) and reduced in a Parr apparatus at 40 psi over 37 h at RT. The catalyst was then removed by filtration and the solvent removed by rotary evaporation (bath at 60°) to give 20.91 g of 2-amino-2-methyl-1,4-butanediol hydrochloride (90.2%) as a clear, thick, colourless oil with acceptable NMR and mass spectra. It was used without further purification. This compound has been reported as its acetate salt (G. Cardillo et al., Chem. Comm. 1308, 1982), but no data was given.

## Example AF

10-Chloroanthracene-1-carboxylic acid

1-Anthroic acid (24 g, 0.108 mol) was treated with N-chlorosuccinimide (Aldrich, 24 g, 0.18 mol) in N-methylpyrrolidinone (Eastman, 600 mL) and heated under $N_2$ at 90° of 1.5 h. The reaction mixture was diluted with 3.5 L ($H_2O$ filtered, dried, and the precipitate recrystallized from EtOAc to afford 16.41 g (59%) of 10-chloroanthracene-1-carboxylic acid mp 257—277°, (C, H, Cl).

Ethyl 10-chloroanthracene-1-carboxylate

10-Chloroanthracene-1-carboxylic acid (17.3 g, 0.0674 mol), *con* $H_2SO_4$ (1.0 mL), and abs. EtOH (500 mL) was refluxed for 3 days using 4 Å molecular sieves in a Soxhlet extractor to remove $H_2O$. The solvent was removed and then partitioned between EtOAc and satd. $NaHCO_3$. The solvent was then removed from the organic layer to give 14.86 g (77%) of ethyl 10-chloroanthracene-1-carboxylate, which was used without further purification.

10-Chlor-1-anthracenemethanol

A solution of ethyl 10-chloroanthracene-1-carboxylate (14.86 g, 0.052 mol) in THF (300 mL) was treated with $LiBH_4$ (Alfa-Ventron, 1.14 g, 0.052 mol) and refluxed for 16 h. The reaction mixture was poured into ice water and acidified with HCl to pH2. The solid was filtered, washed with $H_2O$ (500 mL), air dried and then chromatographed on a plug of $SiO_2$ (500 g) using EtOAc as the eluting solvent. The solvent was removed by rotary evaporation to give a solid, which was crystallized from $CCl_4$ to give 10.3 g (81%) of 10-chloro-1-anthracenemethanol mp 138-140°, (C,H,Cl).

10-chloroanthracene-1-carbaldehyde

10-Chloro-1-anthracenemethanol (8.8 g, 0.036 mol) was dissolved in $CH_2Cl_2$ (200 mL) and treated with $BaMnO_4$ (Aldrich, 15 g, 0.059 mol) for 3 days and briefly brought to reflux. The reaction mixture was filtered, and the filtrate reduced to dryness. The residue was chromatographed by preparative HPLC using $PhCH_3$ as the eluting solvent to give 6.0 g (69%) of slightly impure 10-chloroanthracene-1-carboxaldehyde, which was used without further purification.

## Example AG

3-Nitro-2,4-pentanediol

A solution of nitromethane (Aldrich, 73.3 g, 1.2 mol) and acetaldehyde (Eastman 158.6 g, 3.6 mol) was cooled in an ice bath. $H_2O$ (80 mL) and $Ca(OH)_2$ (0.40 g) were then added to the flask. The mixture was stirred under $N_2$ for 8 h, neutralized with $CO_2$ and filtered. The filtrate was extracted continuously with $CH_2Cl_2$ (1L) for 6 h. The $CH_2Cl_2$ extract was concentrated under vacuum to give 114.6 g (77%) of crude 3-nitro-2,4-pentanediol, a pale yellow syrup. This material was unstable and was used without further purification. Z. Eckstein and T. Urbanski, Rockzniki Chem. 26, 571 (1952), also report the synthesis and isolation of this product as a crude material.

(2α,4α,5α,6α)-4,6-Dimethyl-5-nitro-2-phenyl-1,3-dioxane

A solution of the crude mixture of 3-nitro-2,4-pentanediols (115 g 0.77 mol) from above, benzaldehyde (Fisher 81.7 g, 0.77 mol) and p-toluenesulfonic acid (Fisher 1.28 g) in benzene (400 mL) was refluxed for 1.5 h with azeotropic removal of $H_2O$. After removal of the solvent under vacuum, the crude product (a complex mixture) was dissolved in abs. EtOH (150 mL). After 36 h, the crystals that had formed (RT) were collected and dried to give yield 25.8 g, of a 5:1 mixture (based on NMR) of desired product and another isomer (C,H,N). Pure 2α,4α,5α,6α-4,6-dimethyl-5-nitro-2-phenyl-1,3-dioxane was obtained after recrystallization from abs. EtOH mp 117.5—118° (C,H,N).

meso-3-Amino-2,4-pentanediol acetate

Prepared from (2α,4α,5α,6α)-4,6-dimethyl-5-nitro-2-phenyl-1,3-dioxane as described for example AM except that the temperature was 50°C and subsequently recrystallized from 95% EtOH to give *meso*-3-amino-2,4-pentanediol acetate mp 108.5—109.5°, (C,H,N).

## Example AH

12-Ethyl-6-chrysenecarbaldehyde

6-Ethylchrysene (Cambridge Chemical, Inc. 60 g, 0.234 mol) was formylated according to the procedure outlined in example A, except that $CH_2Cl_2$ (1000 mL) was used as the reaction solvent. The crude material was chromatographed on a plug of $SiO_2$ (1 kg) using $PhCH_3$ as the eluting solvent, affording 50.38 g (76%) of 12-ethyl-6-chrysenecarbaldehyde mp 138—139°, (C,H).

## Example AI

10-(Imidazol-1-yl)-9-anthracenecarbaldehyde

A solution of 10-chloro-9-anthraldehyde (Aldrich, 15 g, 0.062 mol), imidazole (Aldrich, 10.2 g, 0.15 mol) and DMF (300 mL) at 55° was treated with KOtBu (MCB, 7.9 g, 0.07 mol) and stirred for 30 min. The reaction mixture was poured into 0.1M NaOH (1.5 L). The filtered precipitate was chromatographed on a plug of $SiO_2$ (500 g) using $CH_2Cl_2$ (3 L) as the initial eluting solvent to remove starting material and by-products. The yellow product band was then eluted with EtOAc (2 L) to yield (after removal of solvent and drying) 12.29 g (73%) of 10-(imidazol-1-yl)-9-anthracenecarbaldehyde mp 194—196°, (C,H,N), (EtOAc).

## Example AJ

12-Ethoxychrysene-6-carbaldehyde

6-Ethoxychrysene (Cambridge Chemical. Inc., 48 g, 0.176 mol) was formylated according to the procedure outlined in example A, except that $CH_2Cl_2$ (1000 mL) was used as the reaction solvent. After isolation, the crude material was chromatographed on a plug of $SiO_2$ (500 g) using $CH_2Cl_2$ as the eluting solvent to give after removal of solvent and drying 33.7 g (64%) of 12-ethoxychrysene-6-carbaldehyde mp 173.5—176°, (C,H).

## Example AK

4-Chloro-10-(2-hydroxyethoxy)-9-anthracenecarbaldehyde

An isomeric mixture of 1-chloro- and 4-chloro-9-anthraldehydes (36.8 g, 0.133 mol) in ethylene glycol (1000 mL) and THF (200 mL) was treated with KO*t*Bu (MCB, 12.5, 0.11 mol) and heated at 80° for 14 h. The reaction mixture was poured into $H_2O$ (2 L). The precipitate was filtered, washed with $H_2O$ (500 ml) sucked dry, then chromatographed on a plug of silica (500 g) using $CH_2Cl_2$ as the initial eluting solvent to remove starting material and by-products. The desired product was then eluted with EtOAc to give, after removal of solvent and recrystallization from EtOAc, 3.0 g (7.5%) of 4-chloro-10-(2-hydroxyethoxy)-9-anthracenecarbaldehyde mp 141-145°, (C,H,Cl).

## Example AL

Formylation of 3-ethylfluoranthene

3-Ethylfluoranthene (Cambridge Chemical Inc., 70 g, 0.304 mol) was formylated according to the procedure outlined in 1A, except that $CH_2Cl_2$ (1 L) was used as the reaction solvent. Chromatography on a plug of $SiO_2$ (1 kg) yielded three partially purified products, each of which was rigorously purified by preparative HPLC using $PhCH_3$ as the eluting solvent. Each of the three products were isomeric mixtures as described below.

a) 3- and 4-Ethylfluoranthene-7-carbaldehyde, 5.0 g (6%), (Rf=0.55, $SiO_2PhCH_3$),

b) 4-Ethylfluoroanthene-3-carbaldehyde and 3-ethylfluoranthene-2-carbaldehyde, 4.7 g (6%), Rf=0.49, $SiO_2$, $PhCH_3$), (C,H).

c) 3- and 4-Ethylfluoranthene-8-carbaldehyde, 47.3 g (60%), (Rf=0.38, $SiO_2$, $PhCH_3$), (C,H).

d) 4-Ethylfluoranthene-3-carbaldehyde

The mixture b (4.7 g) was recrystallized twice from $CH_2Cl_2$/hexane to yield 1.83 g (2% from 3-ethylfluoranthene) of 4-ethylfluoranthene-3-carbaldehyde mp 113.5—116°, (C,H).

## Example AM

3-Methyl-3-nitro-2,4-pentanediol

Solid NaOH (Mallinckrodt, 286 mg, 7.15 mmol) was added to a solution of 3-nitro-2-butanol (Aldrich, 59.6 g, 0.50 mol) and acetaldehyde (Eastman 132 g, 1.50 mol) in anhydrous DMSO (MCB, 100 mL). The reaction was stirred under $N_2$ for 5 days. Glacial acetic acid (0.5 mL) was then added to the solution. The solvent was then removed by rotary evaporation, (45°C bath temperature) to give a yellow liquid. This was diluted with $H_2O$ (200 mL) and extracted with $CH_2Cl_2$ (5 × 200 mL). The combined $CH_2Cl_2$ extracts were washed sequentially with $H_2O$ (50 mL) and sat. NaCl (50 mL), dried ($MgSO_4$) and filtered. Volatile components were removed from the filtrate under vacuum (first at aspirator vacuum and at 0.1 mm (bath temperature of 50—135°)) leaving a viscous yellow liquid (53.0 g, 64%). This was mixed with EtOAc/hexane (1:1) (50 mL) and subjected to flash chromatography on $SiO_2$ (1.5 kg, Merck silica gel 60 230—400 mesh) using 11 L of EtOAc/hexane (1:1) as the eluting solvent and collecting 500 mL fractions. Appropriate fractions were combined and the solvent removed by rotary evaporation to give a total of 43.5 g (53%) of the disastereomeric mixture of 3-methyl-3-nitro-2,4-pentanediols (two meso forms and a d,l pair, easily distinguished by NMR in DMSO-$d^6$).

(+−)-(2R*,3RS,4R*)-3-Nitro-3-methyl-2,4-pentanediol and meso-3-Nitro-3-methyl-2,4-pentanediol

The chromatographic process described above gave partial separation of the diastereomers. The early fraction (500 mL) gave 13.1 g of one of the *meso*-3-nitro-3-methyl-2,4-pentanediols as a colourless solid mp 60—61° (C,H,N). The remaining fractions were combined to give 38.3 g of the isomeric mixture containing both the meso- and d, l-compounds Recrystallization from EtOAc/hexane (300 mL, 2:1) gave 27.8 g of a 4:1 ratio of (+−)-(2R*,3RS,4R*)-3-Nitro-3-methyl-2,4-pentanediol and the other of the *meso*-3-nitro-3-methyl-2,4-pentanediols mp 79—86° (C,H,N). These two materials were then used withut further purification.

(+−)-(2R*,3RS,4R*)-3-Amino-3-methyl-2,4-pentanediol acetate

To a solution of 3-methyl-3-nitro-2,4-pentanediol (16.3 g, 0.1 mol; the 4:1 mixture of d,l pair of one *meso* form described above) in 95% EtOH (150 mL) was added glacial acetic acid (19 mL) and 10% Pd/C (2.0 g, MCB). The reduction was carried out in a Parr apparatus at 50 psi of $H_2$ during a 70 h period at RT, the catalyst was removed by filtration through a Millipore (TM) filter and the solvent was removed under vacuum (RT, 2 days). The viscous, colourless syrup was dissolved in abs. EtOH (30 mL). While slightly warm, the solution was made cloudy by adding anhydrous $Et_2O$ (100 mL) and was then placed in a refrigerator. Colourless crystals formed over two days which were filtered, washed with $Et_2O$ and dried in a vacuum oven (at RT). The yield of pure (+−)-(2R*,3RS,4R*)-3-amino-3-methyl-2,4-pentanediol acetate (as shown by NMR in DMSO-$d_6$) was 12.8 g mp 110.5-112° (C,H,N). USSR patent 521,272 (CA *85*: 177498) mentions 3-amino-3-methyl-2,4-pentanediol as an intermediate but no synthetic details, physical properties, or stereochemistry was presented in the abstract.

meso-3-Amino-3-methyl-2,4-pentanediol acetate

Using the procedure described above meso-3-methyl-3-nitro-2,4-pentanediol-(undermined configuration) gave meso-3-amino-3-methyl-2,4-pentanediol acetate (53%), mp 137—138°, (C,H,N).

Example AN

(+−) (2R*,3S*)-2-Methyl-2-nitro-1,3-butanediol (A) and (+−) (2R*,3R*)-2-Methyl-2-nitro-1,3-butanediol (B)

To a mixture of 2-nitro-1-propanol (Aldrich, 63.0 g, 0.60 mol) and acetaldehyde (Eastman, 39.6 g, 0.90 mol) cooled in an ice bath under $N_2$ was added cold $H_2O$ (40 mL) and calcium hydroxide (200 mg). The mixture was allowed to warm to RT over 2 h and then stirred for 68 h. The resulting solution was neutralized with excess solid $CO_2$. The mixture was stirred for 1 h before filtration through a Millipore (TM) filter. The filtrate was then concentrated under vacuum at 35°. The residue, a viscous syrup which partially crystallized on drying under vacuum (0.1 mm, RT, 48 h), was triturated with cold $Et_2O$ (35 mL). Solid white crystals which formed were collected by filtration, washed with cold $Et_2O$ (3 × 15 mL) and dried under vacuum (0.1 mm, RT) to give 34.1 g of material, judged by NMR to be diastereomer *A* (purity <97%, racemic). After recrystallization, the diastereomeric purity was >99%, mp 78.5—81° (lit. 78°; cf. Beil 1, 482 in Henry, Bull.Soc.Chim.Fr.[3] 15, 1224), (C,H,N).

The original filtrate (including wash) was concentrated under vacuum to a pale yellow liquid which was subjected to flash chromatography as follows: The sample was mixed with hexane: EtOAc (2:1, 100 mL) and added to a column of dry silica gel 60 (1500 g, Merck, 230—400 mesh). The column was eluted with hexane: EtOAc (2:1, 12 L) then hexane: EtOAc (1:1, 6 L) while 500 mL fractions were collected. Appropriate fractions were combined. Pure product was found in the final 8 L; yield 38.7 g of viscous syrup, judged by NMR to be a 1:1 mixture of the two racemic diaasteromers (*A* and *B*), (C,H,N).

This and another batch of the 1:1 diastereomeric mixture (prepared as described above) were combined (67 g, total) and subjected to successive liquid-liquid partitioning between $H_2O$ and EtOAc to give pure samples (99% on the basis of NMR and HPLC (Hamilton PRP-1 column using 3.5% aqueous acetonitrile as the mobile phase)) of *A* (24.9 g, k'=4.3, C,H,N) and *B* (15.8 g, k'=2.1, C,H,N, a colourless, viscous liquid).

(+−)(2R*,4S*,5R*)-4,5-dimethyl-5-nitro-2-phenyl-1,3-dioxane and (+−)(2R*,4S*,5S*)-4,5-dimethyl-5-nitro-2-phenyl-1,3-dioxane

The relative configurations of the two diastereomeric pairs (*A* and *B*) were assigned on the basis of comparative NMR analysis of the respective cyclic acetals derived from benzaldehyde. Thus, *A* (1.49 g, 0.01 mol) and benzaldehyde (Mallinckrodt, 1.06 g, 0.01 mol) were condensed in benzene in the presence of a catalytic amount of p-toluenesulfonic acid with azeotropic removal of water (according to the method of H. Piotrowska, B. Serafin and T. Urbanski, Tetrahedron *109*, 379 (1963)). After successive washing with sat. $NaHCO_3$ solution, drying ($MgSO_4$), filtration, and removal of the benzene by rotary evaporation, a pale yellow solid was obtained. A solution of this product in ethanol at 0°C provided an oil which was isolated by decanting the mother liquor and drying under vacuum (0.1 mm, RT). The yield was 1.48 g (62%) of (+−)(2R*,4S*,5R*)-4,5-dimethyl-5-nitro-2-phenyl-1,3-dioxane (C,H,N).

Similarly prepared from B and benzaldehyde was (+−)(2R*,4S*,5S*)-4,5-dimethyl-5-nitro-2-phenyl-1,3-dioxane (74%) (C,H,N).

(+−)(2R*,3R*)-2-Amino-2-methyl-1,3-butanediol acetate
Prepared from (+−)(2R*,3R*)-2-methyl-2-nitro-1,3-butanediol as decribed for example AM (97%) (C,H,N) mp 117-121°.

(+−)(2R*,3S*)-2-Amino-2-methyl-1,3-butanediol acetate
Prepared from (+−)(2R*,3S*)-2-methyl-2-nitro-1,3-butanediol as described for AM (93%) (C,H,N) mp 163—165°C.

(+−)(2R*,3R*)-2-Amino-2-methyl-1,3-butanediol acetate
Prepared from (+−)(2R*,3R*)-2-methyl-2-nitro-1,3-butanediol as described for example AM (97%) C,H,N) mp 117-121°.

(+−)(2R*,3S*)-2-Amino-2-methyl-1,3-butanediol acetate
Prepared from (+−)(2R*,3S*)-2-methyl-2-nitro-1,3-butanediol as described for AM (93%) (C,H,N) mp 163-165°C.

Example 1

B. 2-((6-Chrysenylmethyl)amino)-2-methyl-1,3-propanediol hydrochloride

To a 2 L Erylenmeyer flask was added 6-chrysenecarbaldehyde (21.2 g, 82.7 mmol) from example A, 2-methyl-2-amino-1,3-propanediol (Aldrich, 9.13 g, 86.8 mmol), p-toluenesulfonic acid. $H_2O$ (Eastman, 0.5 g, 2.5 mmol), and 500 mL of toluene. The mixture was warmed to reflux for a few minutes and $H_2O$ (2—3 mL) was driven off. The resulting golden coloured solution was allowed to cool to room temperature, diluted with 500 mL of absolute EtOH and stirred overnight. $NaBH_3CN$ (Aldrich, 95%, 2.51 g, 42 mmol) was added to the reaction. After it was dissolved, an indicator (bromocresol green, Eastman, 5 mg) was added. To the resulting blue solution was added 5 drops of 1M g-HCl in absolute EtOH every 15 minutes. After 3 days the indicator turned green then yellow and voluminous white precipitate was present in the flask. To the flask was then added 10 mL of 1M g-HCl in absolute EtOH. The reaction was diluted to 4 with absolute ether and stirred for 1 hour. The precipitate was then filtered through a medium porosity glass fritted funnel and pressed dry. The filter cake was washed thoroughly with 5 × 250 mL portions of 20% HCl, pressed dry and then washed with 4 × 500 mL portions of $CH_2Cl_2$, pressed and sucked dry. The solid was dissolved in 1400 mL of absolute EtOH. 1 mL of 1M g-HCl in absolute EtOH and 5 g of Calgon (Trade Mark) brand of activated charcoal were added and the mixture boiled and filtered through a pad of Celite (Trade Mark of John Manville Co.) brand of filter-aid. The clear yellow solution was concentrated to 500 mL and diluted to 2 with abolute $Et_2O$.

Further crystallisation (2×) from $CH_3OH/ET_2O$ mixtures (1/3) gave 18.07 g (57.2%) mp = 241—243° (dec) of 2-((6-chrysenylmethyl)amino)-2-methyl-1,3-propanediol hydrochloride.

Examples 2—49

Using methods analogous to that described in Example 1 and utilising the appropriate aldehyde and aminoalkanol starting materials, the following compounds of formula (I) were prepared in the form of their hydrochloride salts (all compounds analysed correctly for the assigned structures):

| Compound | Ar | $R^1$ | $R^2$ | R.Solv. | M.P.° |
|---|---|---|---|---|---|
| 2 | 10-Cl-9-An | $CH_2OH$ | $CH_3$ | M/EE | 268—269 (d) |
| 3 | 9-An | $CH_2OH$ | $CH_3$ | M/EE | 139—140(d) |
| 4 | 10-SMe-9-An | $CH_2OH$ | $CH_3$ | E/EE | 225—226(d) |
| 5 | 10-(2-$CH_2CH_2Cl$)-9-An | $CH_2OH$ | $CH_3$ | E/EE | 229—231(d) |
| 6 | 4,10-Cl-9-An | $CH_2OH$ | $CH_3$ | E/EE | 261—262(d) |
| 7 1/4$H_2O$ | 10-$CH_2OH$-9-An | $CH_2OH$ | $CH_3$ | E/EE | 209—210(d) |
| 8 1/2$H_2O$ | 10-Me-9-An | $CH_2OH$ | $CH_3$ | E/EE | >300(d) |
| 9 | 10-Br-9-An | $CH_2OH$ | $CH_2$ | M/EE | 263—264(d) |
| 10 | 10-Cl-9-An | $CH_2OH$ | $C_2H_5$ | M/EE | 252—254 |
| 11 | 4,5-Cl-9-An | $CH_2OH$ | $CH_3$ | E/EE | 239.5—240.5(d) |
| 12 | 3-Fl | $CH_2OH$ | $CH_3$ | M/EE | 262—265.5(d) |
| 13 | 4-Cl-9-An | $CH_2OH$ | $CH_3$ | E/EE | 225—226(d) |
| 14 | 10-$SOCH_3$-9-An | $CH_2OH$ | $CH_3$ | E/EE | 266—268(d) |
| 15 | 2-Tr | $CH_2OH$ | $CH_3$ | E/EE | 207—208.5(d) |
| 16 | 10-OMe-9-An | $CH_2OH$ | $CH_3$ | E/EE | 173—174(d) |
| 17 | 10-CN-9-An | $CH_2OH$ | $CH_3$ | M/EE | 307—308 |
| 18 | 10-Br-1-An | $CH_2OH$ | $CH_3$ | E/EE | 225—226.5(d) |
| 19 | 1-An | $CH_2OH$ | $CH_3$ | E/EE | 189—191(d) |
| 20a | 2-Cl-9-An | $CH_2OH$ | $CH_3$ | M/EE | 265—266(d) |
| b | 3-Cl-9-An | $CH_2OH$ | $CH_3$ | M/EE | 263—269(d) |
| 21 | 2-SEt-9-An | $CH_2OH$ | $CH_3$ | E/EE | 201—202(d) |
| 22 | 2-$SCH_2CH_2OH$-9-An | $CH_2OH$ | $CH_3$ | E/EE | 199—200(d) |
| 23 | 10-Cl-9-An | $CH_2OH$ | $CH_2OH$ | E/EE | 251—254(d) |
| 24 | 3,10-Cl-9-An | $CH_2OH$ | $CH_3$ | M/EE | 303—304(d) |
| 25 | 2,10-Cl-9-An | $CH_2OH$ | $CH_3$ | M/EE | 305—306(d) |
| 26 | 6-Ch | $CH_2OH$ | $CH_2OH$ | M/EE | 238—239(d) |
| 27 | 6-Ch | $CH_2OH$ | $C_2H_5$ | E/EE | 241—243(d) |
| 28 | 3-Fl | $CH_2OH$ | $CH_2OH$ | M/EE | 240—241(d) |
| 29 | 3-Fl | $CH_2OH$ | $C_2H_5$ | M/EE | 250—252(d) |
| 30 | 10-OEt-9-An | $CH_2OH$ | $CH_3$ | E/EE | 229—230(d) |
| 31 | 7-Fl | $CH_2OH$ | $CH_3$ | M/EE | 204—206(d) |
| 32 1/2 $H_2O$ | 10-$CH_2CHOH$-9An | $CH_2OH$ | $CH_3$ | E/EE | 179—181 (d) |

| Compound | Ar | $R^1$ | $R^2$ | R.Solv. | M.P.° |
|---|---|---|---|---|---|
| 33 | 10-$SO_2CH_3$-9-An | $CH_2OH$ | $CH_3$ | M/EE | 238—239(d) |
| 34 | 3-Cl-9-An | $CH_2OH$ | $CH_3$ | M/EE | 268—289(d) |
| 35 | 2-Et-9-An | $CH_2OH$ | $CH_3$ | E/EE | 203—205(d) |
| | 3-Et-9-An | $CH_2OH$ | $CH_3$ | E/EE | |
| *36 | 6-Ch | $CH_2OH$ | $CH_2OEt$ | M/EE | 230—232(d) |
| 37 | 6-Ch | $CH_2OCH_3$ | $CH_3$ | E/EE | 233—234(d) |
| 38 | 3-Fl | $CH_2OCH_3$ | $CH_3$ | E/EE | 222—223(d) |
| *39 9/20 | 3-Fl | $CH_2OH$ | $CH_2OEt$ | E/EE | 179—180 |
| *40 | 9-An | $CH_2OH$ | $CH_2OEt$ | E/EE | 176.5—178.5 |
| *41 | 6-Ch | $CH_2OH$ | $CH_2OEt$ | M/EE | 280—282(d) |
| *42 | 3-Fl | | | M/EE | 258—260(d) |
| **43 1/3$H_2O$ | 6-Ch | $CH_2OH$ | i-Pr | E/EE | 223—223.5(d) |
| **44 | 3-Fl | $CH_2OH$ | i-Pr | E/EE | 216—217(d) |
| *45 1/3EtOH | 6-Ch | $CH_2CH_2OH$ | $CH_3$ | E/EE | 233—235(d) |
| *46 11/20$H_2O$ | 3-Fl | $CH_2CH_2OH$ | $CH_3$ | E/EE | 210—212(d) |
| 47 1/4$H_2O$ | 10-$CH_2CH_2OCH_3$-9-An | $CH_2OH$ | $CH_3$ | E/EE | 182—183(d) |
| 48 11/20$H_2O$ | 10-1m-9-An | $CH_2OH$ | $CH_3$ | E/EE | 212—215(d) |
| 49 | 9-An | $CH_2OCH_3$ | $CH_3$ | E/EE | 214—215(d) |

In all compounds,
$R^3 = R^4 = H$
In examples 41 and 42,

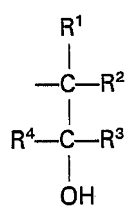

represents a cyclohexanediol ring

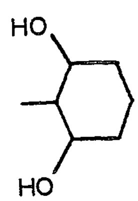

Key: An = anthracenyl
Fl = fluoranthenyl

Tr = triphenylenyl
Ch = chrysenyl
R.Solv = recrystallisation solvent
Im = imidazol-yl
M.P. = melting point
Et = ethyl
i-Pr = *iso*-propyl
M/EE = methanol/diethyl ether
E/EE = ethanol/diethyl ether
(d) = decomposed.

* In these instances the aminoalkanol starting material was in the form of a hydrochloride salt which was neutralised with an equimolar amount of methanolic sodium methoxide and, after warming, the solvent was removed by rotary evaporation before the reductive amination was carried out as described in example 1.
** In these instances the aminoalkanol starting material was in the form of an acetate which was reacted with an equimolar amount of sodium methoxide in methanol and, after warming, the solvent was removed by rotary evaporation before the reductive amination was carried out as described in example 1.

Example 50

A. 2-((6-Chrysenylmethyl)amino)-2-methyl-1,3-propanediol methanesulfonate

To a 12 L round bottomed flask equipped with overhead stirrer, condenser, thermometer, and Dean-Stark trap was added chrysene-6-carbaldehyde (Cambridge Chemical Inc., 202 E. Smith St., Milwaukee, W1, 53207, 260 g, 1.01 mol), 2-amino-2-methyl-1,3-propanediol (Aldrich, 213 g, 2.03 mol), p-toluenesulfonic acid monohydrate (Aldrich, 20.8 g, 0.104 mol) and 3.8 L of $PhCH_3$. The mixture was stirred at reflux with removal of $H_2O$ for 2 h (or until no further $H_2O$ was collected). The mixture was cooled to RT and diluted with 3.8 L of absolute EtOH. Solid sodium borohydride (MCB, 46 g, 1.22 mol) was added in portions to the stirred mixture with the temperature maintained at 25—30° by external cooling. After the addition was completed, the reaction was stirred an additional 3 h at RT. The reaction mixture was then concentrated under vacuum to a volume of 800 mL keeping the flask temperature at 40° or less. The slurry was diluted with $H_2O$ (6 L) and cooled to 5°.

The solid was removed by filtration and washed with $H_2O$ (2 × 1.5 L).† The solid was then suspended in a mixture of SD3A (US Industrial Chem. Co., 2.5 L) and methanesulfonic acid (Alfa Ventron, 107.2 g, 1.12 mol). The resulting solution was filtered and diluted with 5 L of $PhCH_3$. After crystallization overnight at RT the mixture was cooled at 5° for 1 h and filtered. The solid was washed with $PhCH_3$ (100 mL) and dried to give 417 g (93%) (after a second crop obtained from the filtrate was added) of 2((6-Chrysenylmethyl)amino)-2-methyl-1,3-propanediol methanesulfonate mp 239—240° (dec), (C,H,N,S).

B. 2-((6-Chrysenylmethyl)amino)-2-methyl-1,3-propanediol

To a rapidly stirred solution of 2-((6-Chrysenylmethyl)amino)-2-methyl-1,3-propanediol hydrochloride from example 1 (20 g, 52.36 mmol) in a mixture of $CH_3OH$) (200 mL) and $H_2O$ (800 mL) was added dropwise over 10 min a 1M NaOH solution (55 mL). The resulting white precipitate was filtered and washed with warm $H_2O$ (4 × 500 mL) and then with $Et_2O$ (1 L), sucked dry and placed in a vacuum oven overnight. A total of 17.43 g (96.4%) of 2-((6-Chrysenylmethyl)amino)-2-methyl-1,3-propanediol mp 200—202°, (C,H,N) was obtained.

C. 2-((6-Chrysenylmethyl)amino)-2-methyl-1,3-propanediol lactate.

A mixture of 2-((6-Chrysenylmethyl)amino)-2-methyl-1,3-propanediol free base (50B) (3.45 g, 10 mmol) and lactic acid (Fisher, 85% liquid, 1.04 g, 10 mmol) in $CH_3OH$ (500 mL) was brought to reflux and filtered through a glass fritted funnel. The solvent was removed by rotary evaporation to give a crude white solid. This was crystallized ($CH_3OH/Et_2O$) 3 times to give 1.84 g (42.2%) of 2-((6-chrysenylmethyl)amino)-2-methyl-1,3-propanediol lactate mp 163—164°, (C,H,N).

D. 2-((6-Chrysenylmethyl)amino)-2-methyl-1,3-propanediol citrate

A mixture of 2-((6-Chrysenylmethyl)amino)-2-methyl-1,3-propanediol free base (50B) (3.45 g, 10 mmol) and citric acid (Sigma, 1.92, 10 mmol) in $CH_3OH$ (500 mL) was warmed until it dissolved then filtered through a glass fritted funnel. The solvent was then removed to give a crude white solid. This was boiled with abs. EtOH (2 × 300 mL) and filtered to give a white solid. This was then recrystallized 2× ($CH_3OH/Et_2O$) filtered and dried overnight in a vacuum oven to give 1.24 g of 2-((6-Chrysenylmethyl)amino)-2-methyl-1,3-propanediol citrate mp 146—151°, (C,H,N).

---

† Note: In the subsequent procedures referring to this method, the particular example was suspended in either abs. EtOH or $CH_3OH$ then methanesulfonic acid was added. After slight warming and filtration, the resulting solution was diluted with $Et_2O$, hexane, or $PhCH_3$. The precipitate which formed then was filtered and then recrystallized to give the desired compound.

E. 2-((6-Chrysenylmethyl)amino)-2-methyl-1,3-propanediol-hydroxyethanesulfonate

2-((6-Chrysenylmethyl)amino)-2-methyl-1,3-propanediol methanesulfonate (10.0 g, 26.63 mmol) was neutralized with 1N NaOH (30 ml) in $CH_3OH/H_2O$ (200/800 mL) as in procedure 1D. The white solid which formed was filtered, washed successively with warm $H_2O$ (3 × 500 mL), $CH_3OH$ (200 mL) and $Et_2O$ (2 × 500 mL), sucked semidry and then resuspended in $CH_3OH$ (500 mL). To this was added a 0.43 aqueous solution of 2-hydroxyethanesulfonic acid (30 mL). Slight warming gave a solution which was then filtered. The solvent was removed by rotary evaporation to give a wet white solid. This was triturated with dry $Et_2O$ to give 2-((6-Chrysenylmethyl)amino)-2-methyl-1,3-propanediol 2-hydroxyethanesulfonate, (C,H,N,S).

Examples 51—66

Using methods analogous to that described in example 50A, the following compounds of formula (I) were prepared in the form of their methanesulfonate salts (all compounds analysed correctly for the assigned structures):

| Compound | Ar | $R^1$ | $R^2$ | R.Solv. | M.P.° |
|---|---|---|---|---|---|
| 51 | 10-Cl-9-An | $CH_2OH$ | $CH_3$ | M/EE | 234—235(d) |
| 52 | 10-SMe-9-An | $CH_2OH$ | $CH_3$ | E/EE | 193—194(d) |
| 53 | 10-(2-$CH_2CH_2Cl$)-9-An | $CH_2OH$ | $CH_3$ | E/EE | 210—210.5(d) |
| 54 | 4,5-Cl-9-An | $CH_2OH$ | $CH_3$ | M/EE | 252—253(d) |
| 55 | 4-Cl-9-An | $CH_2OH$ | $CH_3$ | M/EE | 223—233.5(d) |
| 56 | 2-Tr | $CH_2OH$ | $CH_3$ | M/EE | 259—261(d) |
| 57 3/4$H_2O$ | 10-morpholino-9-An | $CH_2OH$ | $CH_3$ | E/EE | 159—160(d) |
| 58 | 12-Et-6-Ch | $CH_2OH$ | $CH_3$ | E/EE | 189—192(d) |
| 59 1/3$H_2O$ | 12-Cl-6-CH | $CH_2OH$ | $CH_3$ | E/EE | 233—233.5(d) |
| 60 | 12-$OC_2H_5$-6-Ch | $CH_2OH$ | $CH_3$ | E/EE | 202—204(d) |
| 61 1/3$H_2O$ 1/10 i-PrOH | 4-Cl-10-(2-$OCH_2CH_2OH$)-9-An | $CH_2OH$ | $CH_3$ | P/EE | 156—158(d) |
| 62 | 4-Et-3-Fl | $CH_2OH$ | $CH_3$ | E/hex | 198—199(d) |
| *63 | 6-Ch | $CH_2OH$ | H | M/EE | 208—209(d) |
| *64 | 9-An | $CH_2OH$ | iPr | E/EE | 192—194(d) |
| 65 | 9-An | $CH_2CH_2$ | $OHCH_3$ | E/EE | 212—213(d) |
| 66 | 9-An | see below | | M/EE | 251—252(d) |

* Key as for examples 2 to 49 with the addition that P/EE refers to an isopropanol/trithyl ether solvent mixture and E/hex refers to an ethanol/hexane solvent mixture. In example 66

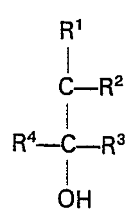

represents a cyclohexanediol-ring

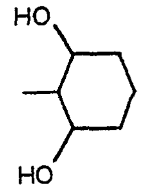

In these instances the aminoalkanol starting material was in the form of a hydrochloride salt which was neutralised with an equimolar amount of methanolic sodium methoxide and, after warming, the solvent was removed by rotary evaporation before the reductive amination was carried out as described in example 1.

Example 67

2-((6-Chrysenylmethyl)amino)-2-methyl-1,3-propanediyl diacetate

A mixture of 2-((6-chrysenylmethyl)amino)-2-methyl-1,3-propanediol hydrochloride (5.0 g, 13.1 mmol) and acetylchloride (Aldrich, 5.0 mL, 70.3 mmol) were refluxed in dry THF (200 mL) under $N_2$ for 12 h. The reaction mixture was poured into saturated $NaHCO_3$ (500 mL) and extracted with EtOAc (3×500 mL). The EtOAc layers were combined, dried ($K_2CO_3$) and filtered to give a slightly yellow clear liquid. The solvent was removed to give an off-white solid. This was recrystallized 3× from $PhCH_3$/hexane (1:1). After filtration and drying, 3.67 g (65.2%) of 2-((6-chrysenylmethyl)amino)-2-methyl-1,3-propanediyl diacetate was obtained mp 136—137.5°, (C,H,N).

Example 68

Meso-3-((6-Chrysenylmethyl)amino)-2,4-pentanediol methanesulfonate

To a round-bottomed flask was added *meso*-3-amino-3-methyl-2,4-pentanediol acetate (57C) and an equimolar amount of sodium methoxide (MCB) and $CH_3OH$ (100 mL). After warming to aid solution, the solvent was removed by rotary evaporation, and after addition of chrysene-6-carbaldehyde the reaction run following the normal reductive amination procedure outlined in example 50A to give *meso*-3(((6-chrysenylmethyl)amino)-2,4-pentanediol methanesulfonate mp 221—223), ($CH_3OH/Et_2O$), (C,H,N,S).

Examples 69 and 70

2-β-((3-Fluoranthenylmethyl)amino)-1-α,3-α-cyclohexandiol methanesulfonate and 2-β-((6-Chrysenylmethyl)amino)-1-α,3-α-cyclohexandiol methanesulfonate

Using the procedure in example 50B, compound 42 was converted to its free base. Addition of an equivalent of methanesulfonic acid (Alfa-Ventron 99.5%) followed by recrystallization ($EtOH/Et_2O$) gave 2-β-((3-fluoranthenylmethyl)amino)-1-α,3-α-cyclohexanediol methanesulfonate, mp 214—216° (d), (C,H,N,S) 2-β-((6-chrysenylmethyl)amino-1-α,3-α-cyclohexanediol methanesulphonate, mp 280—281° (d), (C,H,N,S) was prepared from the corresponding hydrochloride in similar manner.

Example 71

(+−)-(2R*,3RS,4R*)3-((6-Chrysenylmethyl)amino)-3-methyl-2,5-pentanediol methanesulfonate

To a round-bottomed flask was added (+−)-(2R*,3RS,4R*)3-amino-3-methyl-2,4-pentanediol acetate and an equimolar amount of sodium methoxide (MCB) and $CH_3OH$ (100 mL). The solvent was then removed by rotary evaporation and after addition of chrysene-6-carbaldehyde, the reaction run following the normal reductive amination procedure outlined in example 1 to give (+−)-(2R*,3RS,4R*)3-((6-Chrysenylmethyl)amino)-3-methyl-2,5-pentanediol methanesulfonate mp 182—183° (dec). ($EtOH/Et_2O$), (C,H,N,S).

Example 72

(+−)-(2R*,3S*)-2-((6-Chrysenylmethyl)amino)-2-methyl-1,3-butanediolhydrochloride 1/3$H_2O$

To a round-bottomed flask was added (+−)-(*R**,*S**)-2-amino-2-methyl-1,3-butanediol acetate and an equimolar amount of sodium methoxide (MCB) and $CH_3OH$ (100 mL). After warming, the solvent was removed by rotary evaporation, and after addition of chrysene-6-carbaldehyde the reaction run following the normal reductive amination procedure outlined in example 1 to give (+−)(2R*,3S*)-2-((6-chrysenylmethyl)amino)-2-methyl-1,3-butanediol hydrochloride. 1/3 $H_2O$ mp 238—239° (dec), (EtOH $Et_2O$), (C,H,Cl,N).

Example 73

(+−) (2R*,3S*)-2-((9-Anthracenylmethyl)amino)-2-methyl-1,3-butanediolhydrochloride $H_2O$

Following the procedure outlined for example 73 anthracene-9-carbaldehyde (Aldrich) and (+−)-(*R**,*S**)-2-amino-2-methyl-1,3-butanediol acetate gave (+−) (2R*,3S*)-2-((9-Anthracenylmethyl)amino)-2-methyl-1,3-butanediol hydrochloride $H_2O$ mp 216—217° (dec), ($EtOH/Et_2O$), (C,H,Cl,N).

Example 74

(+−)-(2R*,3R*)-2-((6-Chrysenyl)methyl)amino)-2-methyl-1,3-butanediolhydrochloride

Using the procedure outlined for example 73 chrysene-6-carbaldehyde and (+−)(*2R*,3R**)-2-amino-2-methyl-1,3-butanediol acetate (40E) gave (+−)-(2R*,3R*)-2-((6-Chrysenyl)methyl)amino)-2-methyl-1,3-butanediol hydrochloride, mp 236—237.5° (dec), ($CH_3OH/Et_2O$), (C,H,Cl,N).

Example 75

(+−)-(2R*,2S*)-2-(((3-fluoranthenyl)methyl)amino)-2-methyl-1,3-butanediol hydrochloride

Using the procedure outlined for example 73, fluoranthene-3-carbaldehyde and (+−) (2R*,3S*)-2-amino-2-methyl-1,3-butanediol acetate gave (+−)-(2R*,2S*)-2-(((3-fluoranthenyl)methyl)amino)-2-methyl-1,3-butanediol hydrochloride mp 242—243° (dec), $EtOH/Et_2O$, (C,H,Cl,N).

Example 76

(+−) (2R*,3S*)-2-((6-Chrysenylmethyl)amino)-2-methyl-1,3-butanediol methanesulfonate.

Using the reductive amination procedure outlined in example 50A the two intermediates in example 73 gave (+−) (2R*,3S*)-2-((6-Chrysenylmethyl)amino)-2-methyl-1,3-butanediolmethanesulfonate mp 220—221° (dec), ($EtOH/Et_2O$), (C,H,N,S).

Example 77

Antitumour Test Results for 2-((6-chrysenylmethyl)amino)-2-methyl-1,3-propanediol

Methods for evaluating the antitumour activity of these compounds are essentially those used in the Tumour Panel by the Developmental Therapeutics Program, Division of Cancer Treatment, National Cancer Institute, A. Goldin, *et al., Methods in Cancer Research,* Vol. XVI, p. 165, Academic Press (1979). Some modifications, in dose level and schedule have been made to increase the testing efficiency.

Lymphocytic Leukemia P388/0 Test

CD2-$F_1$ mice, of the same sex, weighing within a 3 gram range surrounding 20 g, are used for this test. Control and test animals are injected intraperitoneally with a suspension of $10^6$ viable P388/0 tumor cells on day 0. In each test several dose levels which bracket the $LD_{20}$ for the compound are evaluated; each dose level group contains 6 animals. The test compounds are prepared either in physiologic saline containing 0.05% Tween 80 or distilled water containing 5% dextrose and are administered intraperitoneally on days 1,5 and 9 relative to tumour implant. Doses are on a mg/kg basis according to individual animals' body weights. The day of death for each animal is recorded, the median identified for each group and the ratios of median survival time for treated (T)/control (C) groups are calculated. The criterion for activity is $T/C \times 100 > 120\%$. The results of several tests are summarised in Table I below.

TABLE I

| Compound | Optimal Dosage (mg/kg) | T/C × 100% (Excluding 30 day Survivors) |
|---|---|---|
| 1 | 121 | +280 |
| 3 | 150 | +130 |
| 19 | 77 | +204 |
| 2 | 425 | +228 |
| 9 | 450 | +200 |
| 8 | 94 | +160 |
| 4 | 110 | +262 |
| 5 | 130 | +225 |
| 7 | 165 | +170 |
| 17 | 387 | +190 |
| 14 | 45 | +220 |
| 16 | 120 | +220 |
| 18 | 300 | +225 |
| 6 | 300 | +204 |
| 11 | 300 | +204 |
| 25 | 450 | +210 |
| 24 | 600 | +200 |
| 12 | 90 | +270 |
| 15 | 84 | +200 |
| 13 | 200 | +215 |
| 20a | 150 | +170 |
| 21 | 281 | +145 |
| 22 | 440 | +145 |
| 23 | 277 | +140 |
| 32 | 160 | +300 |

Lymphocytic Leukemia L1210 Test

The protocol for this test is identical with that for P388/0 except that the number of L1210 cells implanted on day 0 is $10^5$/mouse. The mouse strain used is CD2-$F_1$, and the criterion for activity is T/C × 100 >125%. Results of L1210 testing are summarised in Table II below.

TABLE II

| Compound of Example | Optimal Dosage (mg/kg) | T/C × 100% (Excluding 30 Day Survivors) |
|---|---|---|
| 1 | 120 | +252 |
| 4 | 110 | +194 |
| 5 | 150 | +217 |

Malanotic Melanoma B16

B6C3-$F_1$ mice of the same sex, weighing within a 3 gram range surrounding 20 g, are used for this test. A suspension of B16 cells is prepared from a non-necrotic portion of solid tumour tissue obtained from a passage mouse. One gram of tumour is homogenised in 9 mL ice-cold Earle's salts solution and filtered through 100 mesh screen to remove debris. 0.5 mL of the resulting brei is injected intraperitoneally to each animal. Dosing is carried out as in the P388/0 and L1210 tests. Days of death are recorded for a 60 day period and T/C ration calculated as in the P388/0 and L1210 tests. The results of B16 testing are summarised below in Table III.

TABLE III

| Compound of Example | Optimal Dosage (mg/kg) | T/C × 100% (Excluding 30 Day Survivors) |
|---|---|---|
| 1 | 100 | +146 |
| 4 | 110 | +143 |
| 5 | 130 | +146 |
| 6 | 300 | +200 |
| 14 | 30 | +216 |

M5076 Sarcoma Test

This sarcoma arose as a solid tumour in the ovary of a C57B1/6 mouse and was subsequently converted to the ascitic form for intraperitoneal use. The protocol for this test is identical with that for P388/0, the B6C3-$F_1$ mouse strain is used and the criterion for activity is $T/C \times 100 \geq 125\%$. Results of M5076 testing are summarized in Table below.

TABLE
M5076 Screening Data

| Compound | Optimal Dose (mg/kg) | T/C × 100%* |
|---|---|---|
| 1 | 105 | +168 |
| 12 | 85 | + 162 |

* Excluding 30 Day Survivors

Colon 38 Carcinoma Test

This chemically-induced tumour arose in a C57B1/6 mouse and is maintained as a solid tumour in that mouse strain. The subcutaneously growing solid tumour is aseptically excised from passage mice and placed in sterile saline. The tumour is trimmed free of visible necrotic and connective tissue, then divided into 2—3 mm cubes. A cube is implanted subcutaneously in the ventral thoracic region with a sterile tochar on day 0. In each test several dose levels which bracket the $LD_{20}$ for the compound are evaluated. Ten animals are included in each dose level group and 30 in the untreated control group. The test compounds are prepared either in physiologic saline containing 0.05% Tween 80 or distilled water containing 5%

dextrose and are administered intraperitoneally on days 1, 5 and 9 after tumour implant. Doses are on a mg/kg basis according to individual animals' body weights. At day 20 the animals are sacrificed and the longest (L) and the shortest (W) dimensions of each tumour measured with venier calipers. Tumor weight is 100 calculated from the formula L(W)2/2. The criterion for activity is T/C × < 42%. The results of Colon 38 testing are summarized below.

| Compound | Optimal Dose (mg/kg) | T/C × 100% |
|---|---|---|
| 1 | 120 | 36 |
| 5 | 150 | 38 |
| 12 | 65 | 23 |

Lewis Lung Carcinoma Test

This tumour arose spontaneously in the lung of a C57B1/6 mouse and is maintained by subcutaneous passage in the strain. The solid tumour is excised aseptically and placed in sterile saline. Pieces of viable tumour tissue are minced finely with scissors and forced through a 200 mesh stainless steel screen to disaggregate the tumour cells into a suspension. $10^6$ viable cells are injected intravenously into the tail vein of BD-F, mice of the same sex weighing 20±3 grams. In each test several dose levels which bracket the $LD_{20}$ for the compound are evaluated. Ten animals are included in each dose level group and 20 in the untreated control group. The test compounds are prepared and administered as in the P388/0 protocol. The day of death for each animal is recorded, the median identified for each group and the ratios of median survival time for treated (T)/control (C) groups are calculated. The criterion for activity is T/C × 100 ≥ 140%. The results of Lewis lung testing are summarized in Table below.

| Compound | Optimal Dosage (mg/kg) | T/C × 100% |
|---|---|---|
| 1 | 105 | +191 |
| 12 | 85 | +222 |

Example 78: Herpes simples I/vero Test

Antiviral testing against *Herpes simplex* I/vero was done using plaque inhibition methods as outlined in P. Collins and D. J. Bauer, Proc. N.Y. Acad. Sci. 284, 49 (1977) and by plaque reduction methods as outlined in P. Collins and D. J. Bauer, *J. Antimicrobial Chemotherapy 3,* Supplement A, 73 (1977). The column headings labelled Score, Toxicity, and Zone of Inhibition refer to the plaque inhibition screen while the $IC_{50}$ heading to the plaque reduction screen.

TABLE

Results of Antiviral Screening Against herpes simplex I/vero

| Compound No. | Score[A] | $IC_{50}$ |
|---|---|---|
| 2 | −4 | 1.60 μM |
| 3 | −3 | |
| 24 | −4 | |
| 74 | −4 | 12 μM |
| 40 | −2 | 23.8 μM |

A. Score: 0 = no inhibition, −1 = 1—25% inhibition, −2 = 26—50% inhibition
−3 = 51—75% inhibition, −4 = 76—100% inhibition.

Example 79: Candida albicans Test

Antifungal testing against Candida albicans (CN 1863) was done with slight modifications using a combination of broth and agar dilution assays as outlined in Laboratory Handbook of Medical Mycology, Chapter 6, pages 441—446, M. R. McGinnis, Academic press, New York, NY, 1980.

TABLE

Results of Antifungal Testing Against Candida albicans (CN 1863)

| Compound No. | MIC (mg/L) |
|---|---|
| 2 | <50 |
| 3 | <50 |
| 1 | 100 |
| 12 | 30 |

Medium: Wellcotest sensitivity test agar plus 7% lysed horse blood

Example 80

Antibacterial Screening

Antibacterial testing against *Mycoplasma smegmatis* (S3264) and *Streptococcus pyogenes* (CN10) was done with slight modifications using standard agar dilution assays as outlined in Manual of Clinical Microbiology Second Ed., E. H. Lenette, E.H. Spaulding and J.P. Truant Eds., American Society for Microbiology, Washington, DC, 1974.

TABLE

Results of Antibacterial Testing Against Streptococcus pyogenes (CN10)

| Compound No | MIC (mg/L) |
|---|---|
| 1 | <10 |

Example 81: Mycoplasma smegmatis Test

Results of Antibacterial Screening Against Mycoplasma smegmatis (53264)

| Compound No. | MIC (mg/L) |
|---|---|
| 3 | <5 |
| 1 | <10 |
| 58 | 10 |

Example 82: Trichomonas vaginalis Test

Antiprotozoal testing against *Trichomonas vaginalis* was done using methods outlined by R. M. Michaels in Advances in *Chemotherapy 3,*, 39—108(1968).

TABLE

Results of Antiprotozoal Testing Against *Trichomonas vaginalis* (in vitro)

| Compound No. (mg/L) | Dose | Result[A] |
|---|---|---|
| 8 | 40 | −4 |
| 7 | 40 | −4 |

(Stenton or Modified Diamond's medium)

A. Screen Code 0 = no inhibition, −1 = 1—25% inhibition, −2 = 26—50% inhibition, −3 = 51—75% inhibition, −4 = 76—100% inhibition.

Example 83: Nippostrongylus brasiliensis Test

Anthelmintic testing against *Nippostrongylus brasiliensis* was done using methods outlined in D.C. Jenkins, R. Armitage, and T. S. Carrington, *Zeitschrift for Parasitenkunde* 63, 261—269 (1980).

TABLE

Results of Anthelmintic Screening Against *Nippostrongylus brasiliensis* (Immature-free living stages)

| Compound of Example No. | MIC (mg/L) |
|---|---|
| 8 | 50 |
| 3 | ≥50 |
| 2 | ≥50 |

Example 84: Eimeria tenella Testing

Antiprotozoal testing against *Eimeria tenella* was done using methods outlined in V.S. Latter and D. Wilson, *Parasitology 79,* 169 (1979).

TABLE XIII

Results of Antiprotozoa Screening Against Eimeria tenella (in vitro)

| Compound of Example No. | Dose (mg/L) | Result[A] |
|---|---|---|
| 12 | 0.31 | .4 |
| 3 | 1.25 | .4 |

A. Screen Code 0 = no inhibition, −1 = 25% inhibition, −2 = 26—50% inhibition, −3 = 51—75% inhibition, −4 = 76—100% inhibition.

Example 85: $LD_{50}$Tests

TABLE

$LD_{50}$ Values for Selected Compounds
(IP single dose — CD — 1 Male Mouse)

| Compound No. | $LS_{50}$ (mg/kg) |
|---|---|
| 12 | 82 |
| 1 | 140 |
| 4 | 100 |
| 3 | 160 |
| 2 | 250 |
| 32 | 110 |

Example 86: Formulation Examples

A. TABLET

| | |
|---|---|
| Compound of Formula I (as hydrochloride) | 500.0 mg |
| Pregelatinised Corn Starch | 60.0 mg |
| Sodium Starch Glycolate | 36.0 mg |
| Magnesium Stearate | 4.0 mg |

The Compound of the formula (I) is finely ground and intimately mixed with the powdered excipients, pregelatinised corn starch and sodium starch glycolate. The powders are wetted with purified water to form granules. The granules are dried and mixed with the magnesium stearate. The formulation is then compressed into tablets weighing approximately 600 mg each.

B. TABLET

| | |
|---|---|
| Compound of formula (I) | 500.0 mg |
| Corn Starch | 70.0 mg |
| Lactose | 83.8 mg |
| Magnesium Stearate | 4.2 mg |
| Polyvinylpyrrolidone | 14.0 mg |
| Stearic Acid | 28.0 mg |

The Compound of formula (I) is finely ground and intimately mixed with the powdered excipients, corn starch and lactose. The powders are wetted with a solution of polyvinylpyrrolidone dissolved in purified water and denatured alcohol to form granules. The granules are dried and mixed with the powdered stearic acid and magnesium stearate. The formulation is then compressed into tablets weighing approximately 700 mg each.

C. CAPSULES

| | |
|---|---|
| Compound of formula (I) | 500.0 mg |
| Corn Starch | 50.0 mg |
| Magnesium Stearate | 3.0 mg |

The finely divided Compound of formula (I) is mixed with powdered corn starch and wetted with denatured alcohol to densify the powder. The dried powder is mixed with stearic acid and filled into hard-shell gelatin capsules.

D. SYRUP

| | | |
|---|---|---|
| Compound of formula (I) | | 250.0 mg |
| Ethanol | | 250.5 mg |
| Glycerin | | 500.0 mg |
| Sucrose | | 3,500.0 mg |
| Flavouring Agent | | q.s. |
| Colouring Agent | | q.s. |
| Preserving Agent | | 0.1% |
| Purifed Water | q.s. to | 5.0 ml |

The Compound of formula (I) is dissolved in the ethanol, glycerin, and a portion of the purified water. The sucrose and preserving agent are dissolved in another portion of hot purified water, and then the colouring agent is added and dissolved. The two solutions are mixed and cooled before the flavouring agent is added. Purified water is added to final volume. The resulting syrup is thoroughly mixed.

E. IV INJECTION

| | |
|---|---|
| Compound of formula (I) | 5.0 mg |
| Glycerin | q.s. for isotonicity |
| Preservative | 0.1% |
| Hydrochloric Acid or | as needed for |
| Sodium Hydroxide | pH adjustment |
| Water for Injection | q.s. to 1 ml |

The compound of formula (I) and preservative is added to the glycerin and a portion of the water for injection. The pH is adjusted with hydrochloric acid or sodium hydroxide. Water for injection is added to final volume and solution is complete after thorough mixing. The solution is sterilised by filtration through a 0.22 micrometer membrane filter and aseptically filled into sterile 10 ml ampoules or vials.

**Claims**

1. A compound of the formula (I):

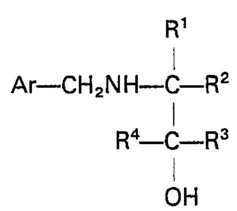

(I)

or a monomethyl or monethyl ether thereof, the compound of formula (I) includings its ethers containing not more than 28 carbon atoms in total, or an ester of salt thereof;

wherein Ar is selected from the group comprising:

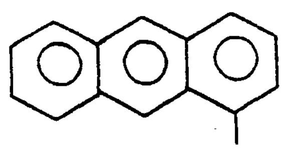 , 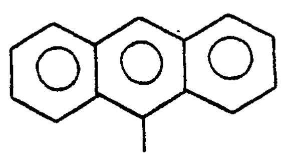 , 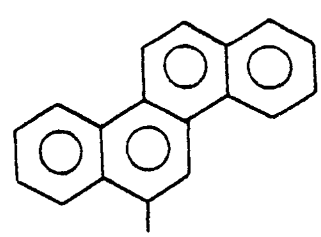 ,

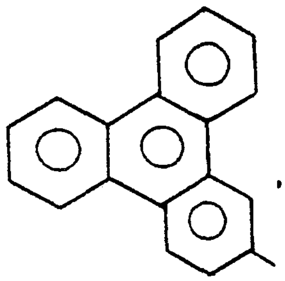,
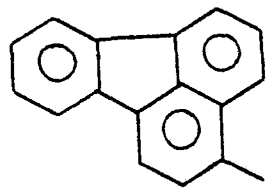
and
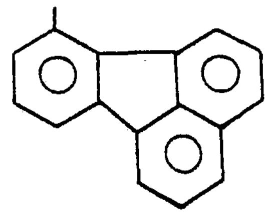

optionally substituted by one or two substituents which taken together contain not more than four carbon atoms in total and which are the same or different and are selected from halo; cyano; $C_{1-3}$ alkyl or $C_{1-3}$ alkoxy each optionally substituted by hydroxy or $C_{1-2}$ alkoxy; halo substituted $C_{1-2}$ alkyl or $C_{1-2}$ alkoxy; a group $S(O)_nR^5$ wherein n is an integer 0, 1 or 2 and $R^5$ is $C_{1-2}$ alkyl optionally substituted by hydroxy or $C_{1-2}$ alkoxy; or Ar is optionally substituted by a group $NR^6R^7$ containing not more than 5 carbon atoms wherein $R^6$ and $R^7$ are the same or different and each is a $C_{1-3}$ alkyl group or $NR^6R^7$ forms a five or six membered heterocyclic ring optionally containing one or two additional hetero atoms;

$R^1$ is $C_{1-3}$ alkyl substituted by hydroxy;

$R^2$ is hydrogen, $C_{1-3}$ alkyl or hydroxymethyl;

$R^3$ and $R^4$ are the same or different and each is hydrogen, methyl or ethyl; $R^1$, $R^2$, $R^3$ and $R^4$ taken together containing not more than five carbon atoms;

or the group:

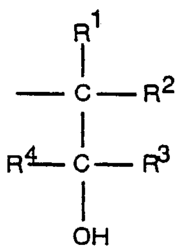
is
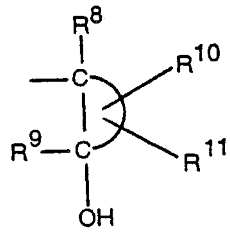

wherein

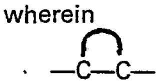 is a five or six membered saturated carbocyclic ring containing two or three hydroxy groups;

$R^8$ is hydrogen, methyl or hydroxymethyl;

$R^9$ and $R^{10}$ are the same or different and each is hydrogen or methyl;

$R^{11}$ is hydrogen, hydroxy, methyl or hydroxymethyl;

$R^8$, $R^9$, $R^{10}$, $R^{11}$ and the 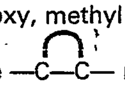 ring taken together containing less than seven carbon atoms.

2. A compound according to claim 1 wherein Ar is 6-chyrsenyl, 7-fluoranthenyl or substituted 1- or 9-anthracenyl

3. A compound according to either claim 1 or 2 wherein Ar is substituted by $C_{1-2}$ alkyl or $C_{1-2}$ alkoxyl each optionally substituted by chloro, hydroxy or methoxy; or a group $(S(O)_nR^5$ or chloro, imidazolyl, morpholino, cyano or bromo.

4. A compound according to any one of claims 1 to 3 in which

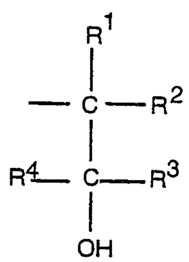
is
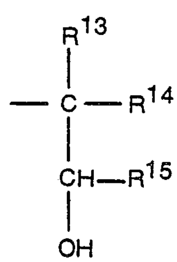
or
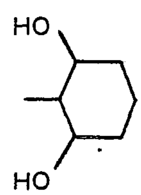

wherein

$R^{13}$ is $CH_2OH$, $CH(CH_3)OH$ or $CH_2CH_2OH$;

$R^{14}$ is hydrogen, $C_{1-3}$ alkyl, or $CH_2OH$;

$R^{15}$ is hydrogen or methyl.

5. A compound according to any one of claims 1 to 4 in which

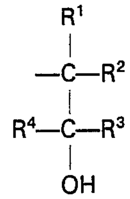
is
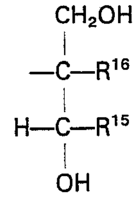

wherein $R^{15}$ is hydrogen or methyl and $R^{16}$ is hydrogen, methyl or ethyl.

6. A compound of the formula (I) according to claim 1 selected from the group comprising:
2-((6-Chrysenylmethyl)amino)-2-methyl-1,3-propanediol,
2-((9-Anthracenylmethyl)amino)-2-methyl-1,3-propanediol,
2-((1-Anthracenylmethyl)amino)-2-methyl-1,3-propanediol,
2-((10-Chloro-9-anthracenylmethyl)-amino)-2-methyl-1,3-propanediol,
2-((10-Bromo-9-anthracenylmethyl)amino)-2-methyl-1,3-propanediol,
2-Methyl-2-((10-methyl-9-anthracenylmethyl)amino)-1,3-propanediol,
2-Methyl-2-((10-methylthio-9-anthracenylmethyl)amino)-1,3-propanediol,
2-((10-(2-Chloroethyl)-9-anthracenylmethyl)amino)-2-methyl-1,3-propanediol,
2-((10-Hydroxymethyl)-9-anthracenylmethyl)amino)-2-methyl-1,3-propanediol,
10-((1,1-Bis(hydroxymethyl)ethylamino)methyl-9-anthracene-carbonitrile,
2-Methyl-2-((10-methylsulfinyl-9-anthracenylmethyl)amino)-1,3-propanediol,
2-((10-Methoxy-9-anthracenylmethyl)amino)-2-methyl-1,3-propanediol,
2-((10-Bromo-1-anthracenylmethyl)amino)-2-methyl-1,3-propanediol,
2-((4,10-Dichloro-9-anthracenylmethyl)amino)-2-methyl-1,3-propanediol,
2-((4,5-Dichloro-9-anthracenylmethyl)amino)-2-methyl-1,3-propanediol,
2-((2,10-Dichloro-9-anthracenylmethyl)amino)-2-methyl-1,3-propanediol,
2-((3,10-Dichloro-9-anthracenylmethyl)amino)-2-methyl-1,3-propanediol,
2-Methyl-2-((2-triphenylenylmethyl)amino)-1,3-propanediol,
2-((4-Chloro-9-anthracenylmethyl)amino)-2-methyl-1,3-propanediol,
2-((2-Chloro-9-anthracenylmethyl)amino)-2-methyl-1,3-propanediol,
2-((10-Ethylthio-9-anthracenylmethyl)amino)-2-methyl-1,3-propanediol,
2-((10-(2-Hydroxyethylthio)-9-anthracenylmethyl)amino)-2-methyl-1,3-propanediol,
2-((10-Chloro-9-anthracenylmethyl)amino)-2-hydroxymethyl-1,3-propanediol,
2-((7-Fluoranthenylmethyl)amino)-2-methyl-1,3-propanediol,
2-((10-(2-Hydroxyethyloxy-9-anthracenylmethyl)amino)-2-methyl-1,3-propanediol,
2-((10-Ethoxy-9-anthracenylmethyl)amino)-2-methyl-1,3-propanediol,
2-((6-Chrysenylmethyl)amino)-2-hydroxymethyl-1,3-propanediol,
2-((6-Chrysenylmethyl)amino)-2-ethyl-1,3-propanediol,
2-Hydroxymethyl-2-((3-fluoranthenylmethyl)amino)-1,3-propanediol,
2-Ethyl-2-((3-fluoranthenylmethyl)amino)-1,3-propanediol,
2-((10-chloro-9-anthracenylmethyl)amino)-2-ethyl-1,3-propanediol,
2-((3-chloro-9-anthracenylmethyl)amino)-2-methyl-1,3-propanediol,
(+ −)-(2R*,3S*)-2-((6-chrysenylmethyl)amino)-2-methyl-1,3-butanediol,
2-((2-ethyl-9-anthracenylmethyl)amino)-2-methyl-1,3-propanediol and 2-((3-ethyl-9-anthracenylmethyl)amino)-2-methyl-1,3-propanediol,
(+ −)(2R*,3S*)-2-((9-anthracenylmethyl)amino)-2-methyl-1,3-butanediol,
(+ −)(2R*,3R*)-2-(((6-chrysenyl)methyl)amino)-2-methyl-1,3-butanediol,
2-(((6-chrysenyl)methyl)amino)-2-ethoxymethyl-1,3-propanediol,
3-methoxy-2-(((6-chrysenyl)methyl)amino)-2-methyl-1-propanol,
3-methoxy-2(((3-fluoranthenyl)methyl)amino)-2-methyl-1-propanol,
(+ −)(2R*,2S*)-2-(((3-fluoranthenyl)methyl)amino)-2-methyl-1,3-butanediol,
2-ethoxymethyl-2-(((3-fluoranthenyl)methyl)amino)-1,3-propanediol,
2-(((9-anthracenyl)methyl)amino)-2-ethoxymethyl-1,3-propanediol,
2-β-((6-chrysenylmethyl)amino)-1-α,3-α-cyclohexanediol,
2-β-((3-fluoranthenylmethyl)amino)-1-α,3-α-cyclohexanediol,
2-((6-chrysenylmethyl)amino)-2-isopropyl-1,3-propanediol,
2-((3-fluoranthrenylmethyl)amino)-2-isopropyl-1,3-propanediol,
2-((6-chrysenylmethyl)amino)-2-methyl-1,4-butanediol,
2-((3-fluoranthenylmethyl)amino)-2-methyl-1,4-butanediol,
2-(((10-chloro-1-anthracenyl)methyl)amino)-2-methyl-1,3-propanediol,
Meso-3-((6-chrysenylmethyl)amino)-2,4-pentanediol,
2-((6-chrysenylmethyl)amino)-1,3-propanediol,
2-(((12-ethyl-6-chrysenyl)methyl)amino)-2-methyl-1,3-propanediol,
2-(((10-(2-methoxyethoxy)-9-anthracenyl)methyl)amino)-2-methyl-1,3-propanediol,
2-methyl-2-(((10-morpholino-9-anthracenyl)methyl)amino)-1,3-propanediol,

2-((9-anthracenylmethyl)amino)-3-methoxy-2-methyl-1-propanol,
2-(((12-chloro-6-chrysenyl)methyl)amino)-2-methyl-1,3-propanediol,
2-((9-anthracenylmethyl)amino)-2-isopropyl-1,3-propanediol,
2-((9-anthracenylmethyl)amino)-2-methyl-1,4-butanediol,
2-(((10-(1H-imidazol-1-yl)-9-anthracenyl)methyl)amino)-2-methyl-1,3-propanediol,
2-(4-ethyl-3-fluoranthenyl)methyl)amino)-2-methyl-1,3-propanediol,
2-(((12-ethoxy-6-chrysenyl)methyl)amino)-2-methyl-1,3-propanediol,
(1α,2β,3α)-2-(9-anthracenylmethyl)amino)-1,3-cyclohexanediol
2-(((4-chloro-10-hydroxyethoxy)-9-anthracenyl)methyl)amino)-2-methyl-1,3-propanediol,
(+ −)(2R⁺,/RS⁺, 4R⁺)-3-(6-chrysenylmethyl)amino)-3-methyl-2,5-pentanediol,
2-methyl-2-(((10-methylsulphonyl-1-9-anthracenyl)methyl)amino)-1,3-propanediol,
and salts and esters thereof.

7. 2-((6-chrysenylmethyl)amino)-2-methyl-1,3-propanediol or a salt or ester thereof.

8. Hydrochloride, methansulfonate, ethanesulfonate, lactate, citrate or isethionate salt of a compound of the formula (I) according to any of claims 1 to 8.

9. A pharmaceutical formulation comprising a compound of the formula (I) or an ether, ester or salt thereof according to any one of claims 1 to 8 together with a pharmaceutically acceptable carrier therefor.

10. A pharmaceutical composition according to claim 11, that comprises a sterile aqueous solution of a pharmaceutically and pharmacologically acceptable acid addition salt of a compound of the formula (I) according to any one of claims 1 to 6, that is isotonic with the blood of the recipient.

11. A method for the preparation of a pharmaceutical formulation which comprises bringing into association a compound of formula (I) or an ether, ester or a pharmaceutically acceptable salt thereof and a pharmaceutically acceptable carrier therefor.

12. A method for the preparation of a compound of the formula (I) according to any one of claims 1 to 7, which comprises:

(i) the reduction of a compound of formula (II):

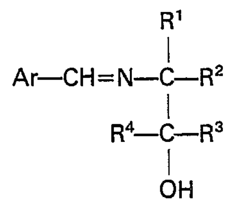

(II)

wherein $R^1$ to $R^4$ are as hereinbefore defined, or an appropriately protected derivative thereof, followed by deprotection where appropriate;

(ii) the reduction of a compound of the formula (V):

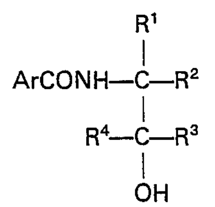

(V)

wherein $R^1$ to $R^4$ are as hereinbefore defined and the hydroxy groups are optionally protected, followed by deprotecton of the hydroxy groups where appropriate, or

(iii) the reaction of a compound $ArCH_2L$, wherein Ar is as hereinbebore defined and L is a leaving group, with a compound of the formula (IV):

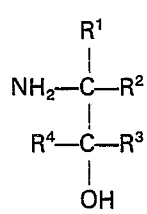

wherein $R^1$ to $R^4$ are as hereinbefore defined.

13. A chemical intermediate of the following formula (II)

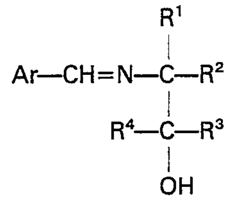

(II)

wherein Ar and $R^1$, $R^2$, $R^3$ and $R^4$ are as defined in claim 1.

14. A chemical intermediate of the following formula (V)

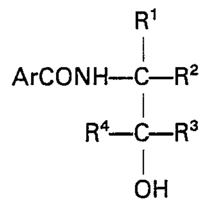

(V)

wherein Ar, $R^1$, $R^2$, $R^3$, $R^4$ are as defined in claim 1.

15. 2(((10-(2-hydroxyethoxy)-9-anthracenyl)methyl)amino)-2-methyl-1,3-propanediol or a salt or ester thereof.

16. 2((3-fluroanthenylmethyl)amino)-2-methyl-1,3-propanediol or a salt or ester thereof.

**Patentansprüche**

1. Verbindung der Formel (I):

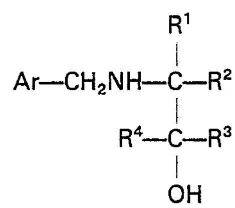

(I)

oder deren Monomethyl- oder Monoethyleter, wobei die Verbinding der Formel (I) einschließlich ihrer Ether nicht mehr als insgesamt 238 Kohlenstoffatome enthält, oder ein Ester oder ein Salz davon:

worin Ar aus der folgenden Gruppe ausgewählt ist:

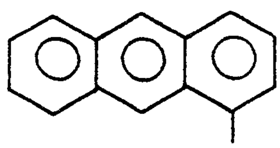 , 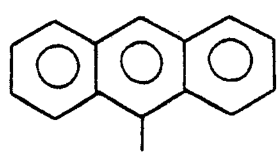 , 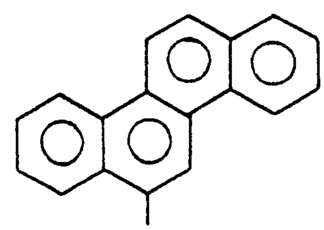 ,

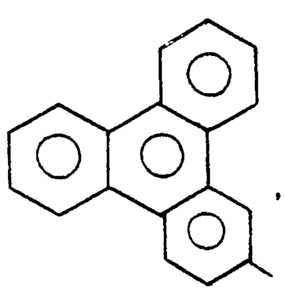 , 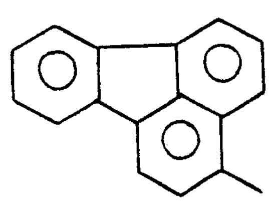 und 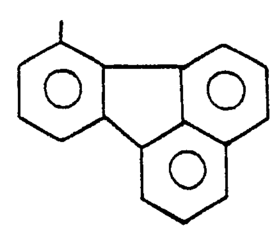

wobei die Reste gegebenenfalls durch einen oder zwei Substituenten, die gleich oder verschieden sind und zusammengenommen nicht mehr als insgesamt 4 Kohlenstoffatome enthalten substituiert sind und wobei

diese aus der folgenden Gruppe ausgewählt sind: Halogen, Cyano, $C_{1-3}$-Alkyl oder $C_{1-3}$-Alkoxy, wobei jeder Rest gegebenenfalls durch Hydroxy oder $C_{1-2}$-Alkoxy substituiert ist; Halogen substituiertes $C_{1-2}$-Alkyl oder $C_{1-2}$-Alkoxy; eine Gruppe $S(O)_nR^5$ worin n eine ganze Zahl 0, 1 oder 2 bedeutet und $R^5$ ein gegebenenfalls durch Hydroxy oder $C_{1-2}$-Alkoxy substituiertes $C_{1-2}$-Alkyl bedeutet; oder Ar ist gegebenenfalls durch eine Gruppe $NR^6R^7$, die nicht mehr als 5 Kohlenstoffatome enthält, substituiert, worin $R^6$ und $R^7$ gleich oder verschieden sein können und jeder Rest eine $C_{1-3}$-Alkylgruppe bedeutet oder $NR^6R^7$ bildet einen 5- oder 6-gliedriegen heterocyclischen Ring, der gegebenenfalls eines oder zwei zusätzliche Heteroatome enthält; und

$R^1$ ein durch Hydroxy substituiertes $C_{1-3}$-Alkyl bedeutet, und

$R^2$ Wasserstoff, $C_{1-3}$-Alkyl oder Hydroxymethyl bedeutet;

$R^3$ und $R^4$, die gleich oder verschieden sind bedeuten Wasserstoff, Methyl oder Ethyl;

$R^1$, $R^2$, $R^3$ und $R^4$ enthalten zusammengenommen nicht mehr als 5 Kohlenstoffatome;

oder der Rest:

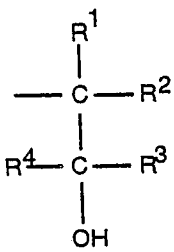 bedeutet den Rest 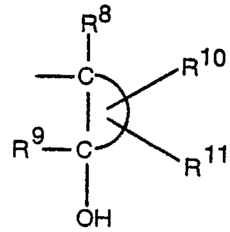

worin 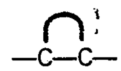 einen fünf oder sechsgliedrigen gesättigten carbocyclischen Ring, der zwei oder drei Hydroxylgruppen enthält bedeutet;

$R^8$ Wasserstoff, Methyl oder Hydroxymethyl bedeutet;

$R^9$ und $R^{10}$, die gleich oder verschieden sind, bedeuten jeweils Wasserstoff oder Methyl;

$R^{11}$ bedeutet Wasserstoff, Hydroxyl, Methyl oder Hydroxymethyl;

$R^8$, $R^9$, $R^{10}$, $R^{11}$ und der 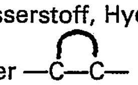 Ring ethalten zusammengenommen weniger als 7 Kohlenstoffatome.

2. Verbindung nach Anspruch 1, worin Ar 6-Chyrsenyl, 7-Fluoranthenyl oder substituiertes 1- oder 9-Anthracenyl bedeutet.

3. Verbindung nach Anspruch 1 oder 2, worin Ar durch $C_{1-2}$-Alkyl oder $C_{1-2}$-Alkoxyl substituiert ist und wobei jeder Rest gegebenenfalls durch Chlor, Hydroxy oder Methoxy; ein Gruppe $S(O)_nR^5$, Chlor, Imidazolyl, Morpholino, Cyano oder Brom substituiert ist.

4. Verbindung nach einem der Ansprüche 1 bis 3, in der

den Rest 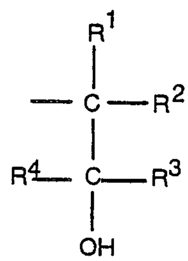 den Rest 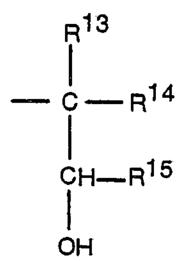 oder 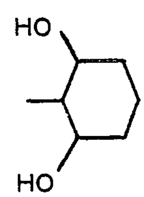

bedeutet, worin

$R^{13}$ $CH_2OH$, $CH(CH_3)OH$ oder $CH_2CH_2OH$ bedeutet;

$R^{14}$ Wasserstoff, $C_{1-3}$-Alkyl oder $CH_2OH$ bedeutet; und

$R^{15}$ Wasserstoff oder Methyl bedeutet.

5. Verbindung nach einem der Ansprüche 1 bis 4, worin der Rest

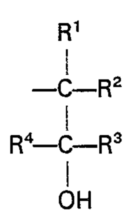 den Rest 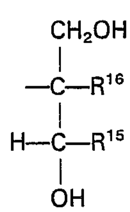 bedeutet

worin $R^{15}$ Wasserstoff oder Methyl und $R^{16}$ Wasserstoff, Methyl oder Ethyl bedeutet.

6. Verbindung der Formel (I) nach Anspruch 1 ausgewählt aus der folgenden Gruppe:

2-((6-Chrysenylmethyl)amino)-2-methyl-1,3-propandiol,

2-((9-Anthracenylmethyl)amino)-2-methyl-1,3-propandiol,
2-((1-Anthracenylmethyl)amino)-2-methyl-1,3-propandiol,
2-((10-Chlor-9-anthracenylmethyl)-amino)-2-methyl-1,3-propandiol,
2-((10-Brom-9-anthracenylmethyl)amino)-2-methyl-1,3-propandiol,
2-Methyl-2-((10-methyl-9-anthracenylmethyl)amino)-1,3-propandiol,
2-Methyl-2-((10-methylthio-9-anthracenylmethyl)amino)-1,3-propandiol,
2-((10-(2-Chlorethyl)-9-anthracenylmethyl)amino)-2-methyl-1,3-propandiol,
2-((10-Hydroxymethyl)-9-anthracenylmethyl)amino)-2-methyl-1,3-propandiol,
10-((1,1-Bis(hydroxymethyl)ethylamino)methyl-9-anthracen-carbonitril,
2-Methyl-2-((10-methylsulfinyl-9-anthracenylmethyl)amino)-1,3-propandiol,
2-((10-Methoxy-9-anthracenylmethyl)amino)-2-methyl-1,3-propandiol,
2-((10-Brom-1-anthracenylmethyl)amino)-2-methyl-1,3-propandiol,
2-((4,10-Dichlor-9-anthracenylmethyl)amino)-2-methyl-1,3-propandiol,
2-((4,5-Dichlor-9-anthracenylmethyl)amino)-2-methyl-1,3-propandiol,
2-((2,10-Dichlor-9-anthracenylmethyl)amino)-2-methyl-1,3-propandiol,
2-((3,10-Dichlor-9-anthracenylmethyl)amino)-2-methyl-1,3-propandiol,
2-Methyl-2-((2-triphenylenylmethyl)amino)-1,3-propandiol,
2-((4-Chlor-9-anthracenylmethyl)amino)-2-methyl-1,3-propandiol,
2-((2-Chlor-9-anthracenylmethyl)amino)-2-methyl-1,3-propandiol,
2-((10-Ethylthio-9-anthracenylmethyl)amino)-2-methyl-1,3-propandiol,
2-((10-(2-Hydroxyethylthio)-9-anthracenylmethyl)amino)-2-methyl-1,3-propandiol,
2-((10-Chlor-9-anthracenylmethyl)amino)-2-hydroxymethyl-1,3-propandiol,
2-((7-Fluoranthenylmethyl)amino)-2-methyl-1,3-propandiol,
2-((10-(2-Hydroxyethyloxy)-9-anthracenylmethyl)amino-2-methyl-1,3-propandiol,
2-((10-Ethoxy-9-anthracenylmethyl)amino)-2-methyl-1,3-propandiol,
2-((6-Chrysenylmethyl)amino)-2-hydroxymethyl-1,3-propandiol,
2-((6-Chrysenylmethyl)amino)-2-ethyl-1,3-propandiol,
2-Hydroxymethyl-2-((3-fluoranthenylmethyl)amino)-1,3-propandiol,
2-Ethyl-2-((3-fluoranthenylmethyl)amino)-1,3-propandiol,
2-((10-chlor-9-anthracenylmethyl)amino)-2-ethyl-1,3-propandiol,
2-((3-chlor-9-anthracenylmethyl)amino)-2-methyl-1,3-propandiol,
(+ −)-(2R*,3S*)-2-((6-Chrysenylmethyl)amino)-2-methyl-1,3-butandiol,
2-((2-Ethyl-9-anthracenylmethyl)amino)-2-methyl-1,3-propandiol und 2-((3-Ethyl-9-anthracenylmethyl)amino)-2-methyl-1,3-propandiol,
(+ −)(2R*,3S*)-2-((9-Anthracenylmethyl)amino)-2-methyl-1,3-butandiol,
(+ −)(2R*,3R*)-2-(((6-Chrysenyl)methyl)amino)-2-methyl-1,3-butandiol,
2-(((6-Chrysenyl)methyl)amino)-2-ethoxymethyl-1,3-propandiol,
3-Methoxy-2-(((6-chrysenyl)methyl)amino)-2-methyl-1-propanol,
3-Methoxy-2(((3-fluoranthenyl)methyl)amino)-2-methyl-1-propanol,
(+ −)(2R*,2S*)-2-(((3-Fluoranthenyl)methyl)amino)-2-methyl-1,3-butandiol,
2-Ethoxymethyl-2-(((3-fluoranthenyl)methyl)amino)-1,3-propandiol,
2-(((9-Anthracenyl)methyl)amino)-2-ethoxymethyl-1,3-propandiol,
2-β-((6-Chrysenylmethyl)amino)-1-α,3-α-cyclohexandiol,
2-β-((3-Fluoranthenylmethyl)amino)-1-α,3-α-cyclohexandiol,
2-((6-Chrysenylmethyl)amino)-2-isopropyl-1,3-propandiol,
2-((3-Fluoranthrenylmethyl)amino)-2-isopropyl-1,3-propandiol,
2-((6-Chrysenylmethyl)amino)-2-methyl-1,4-butandiol,
2-((3-Fluoranthenylmethyl)amino)-2-methyl-1,4-butandiol,
2-(((10-Chlor-1-anthracenyl)methyl)amino)-2-methyl-1,3-propandiol,
Meso-3-((6-chrysenylmethyl)amino)-2,4-pentandiol,
2-((6-Chrysenylmethyl)amino)-1,3-propandiol,
2-(((12-Ethyl-6-chrysenyl)methyl)amino)-2-methyl-1,3-propandiol,
2-(((10-(2-Methoxyethoxy)-9-anthracenyl)methyl)amino)-2-methyl-1,3-propandiol,
2-Methyl-2-(((10-morpholino-9-anthracenyl)methyl)amino)-1,3-propandiol,
2-((9-Anthracenylmethyl)amino)-3-methoxy-2-methyl-1-propanol,
2-(((12-Chlor-6-chrysenyl)methyl)amino)-2-methyl-1,3-propandiol,
2-((9-Anthracenylmethyl)amino)-2-isopropyl-1,3-propandiol,
2-((9-Anthracenylmethyl)amino)-2-methyl-1,4-butandiol,
2-(((10-(1H-Imidazol-1-yl)-9-anthracenyl)methyl)amino)-2-methyl-1,3-propandiol,
2-(4-Ethyl-3-fluoranthenyl)methyl)amino)-2-methyl-1,3-propandiol,
2-(((12-Ethoxy-6-chrysenyl)methyl)amino)-2-methyl-1,3-propandiol,
(1α,2β,3α)-2-(9-Anthracenylmethyl)amino)-1,3-cyclohexandiol,
2-(((4-Chlor-10-hydroxyethoxy)-9-anthracenyl)methyl)amino)-2-methyl-1,3-propandiol,
(+ −)(2R$^+$,/RS$^+$, 4R$^+$)-3-(6-Chrysenylmethyl)amino)-3-methyl-2,5-pentandiol,

2-Methyl-2-(((10-methylsulphonyl-1-9-anthracenyl)methyl)amino)-1,3-propandiol, und ihre Salze und Ester.

7. 2-((6-Chrysenylmethyl)amino)-2-methyl-1,3-propandiol oder ein Salz oder ein Ester davon.

8. Das Hydrochlorid, Methansulfonat, Ethansulfonat, Lactat, Citrat oder Isethionatsalz einer Verbindung der Formel (I) nach einem der Ansprüche 1 bis 8.

9. Pharmazeutische Formulierung enthaltend eine Verbindung der Formel (I) oder einen Ether, Ester oder Salz davon, nach einem der Ansprüche 1 bis 8 zusammen mit einem dafür annehmbaren Trägerstoff.

10. Pharmazeutische Zusammensetzung nach Ansprüch 11, die eine sterile wässrige Lösung eines pharmazeutisch und pharmakologisch annehmbaren Säureadditionssalzes einer Verbindung der Formel (I) nach einem der Ansprüche 1 bis 6 enthält, die mit dem Blut des Empfängers isotonisch ist.

11. Verfahren zur Herstellung einer pharmazeutischen Formulierung bei dem man eine Verbindung der Formel (I) oder einen Ether, Ester oder ein pharmazeutisch annehmbares Salz davon mit einem pharmazeutisch annehmbaren Trägerstoff in Verbindung bringt.

12. Verfahren zur Herstellung einer Verbindung der Formel (I) nach einem der Ansprüche 1 bis 7, bei dem man:

(i) eine Verbindung der Formel (II) reduziert

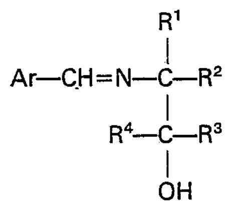

(II)

worin $R^1$ bis $R^4$ die vorhin gegebene Bedeutung aufweisen oder ein entsprechend geschütztes Derivat davon, gefolgt, wo angebracht, von der Entfernung des Schutzes;

(ii) eine Verbindung der Formel (V) reduziert

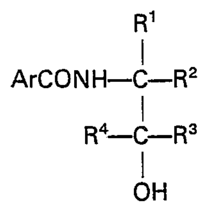

(V)

worin $R^1$ bis $R^4$ die vorhin gegebene Bedeutung aufweisen und die Hydroxygruppen gegebenenfalls geschützt sind, gefolgt, wo angebracht, von der Entfernung des Schutzes von den Hydroxygruppen, oder

(iii) eine Verbindung $ArCH_2L$, worin Ar die vorhin gegehene Bedeutung aufweist und L eine austrehende Gruppe bedeutet mit einer Verbindung der Formel (IV) umsetzt:

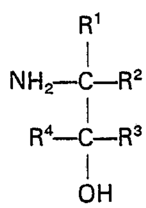

(IV)

worin $R^1$ bis $R^4$ die vorhin gegebene Bedeutung aufweisen.

13. Chemisches Zwischenprodukt der folgenden Formel (II)

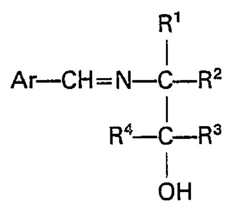

(II)

worin Ar und $R^1$, $R^2$, $R^3$ und $R^4$ die in Anspruch 1 gegebene Bedeutung aufweisen.

14. Chemisches Zwischenprodukt der Formel (V)

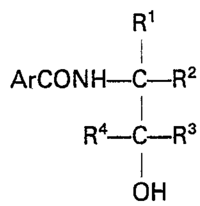

(V)

worin Ar, $R^1$, $R^2$, $R^3$, $R^4$ die in Anspruch 1 gegebene Bedeutung aufweisen.

15. 2(((10-(2-Hydroxyethoxy)-9-anthracenyl)methyl)amino)-2-methyl-1,3-propandiol oder ein Salz oder ein Ester davon.

16. 2((3-Fluroanthenylmethyl)amino)-2-methyl-1,3-propandiol oder ein Salz oder ein Ester davon.

**Revendications**

1. Composé de formule

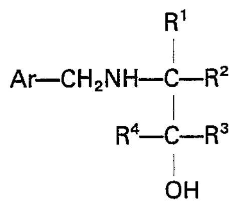

(I)

ou un éther monométhylique ou monoéthylique de celui-ci, le composé de formule (I) comprenant ses éthers ne comptant pas plus de 28 atomes de carbone au total, ou un sel ou ester qui en dérive;

où Ar est choisi parmi:

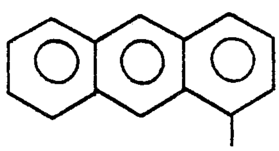
,
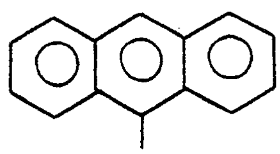
,
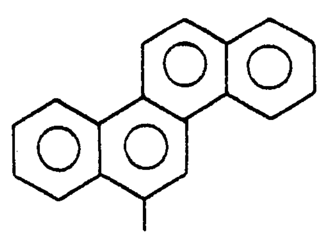
,
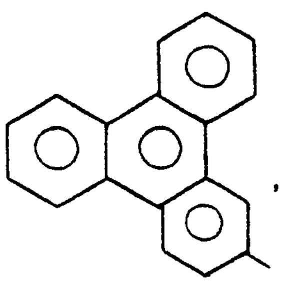
,
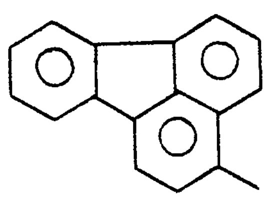
et
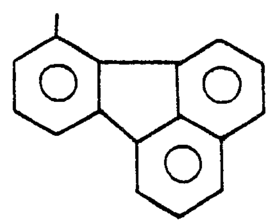

éventuellement substitué par un ou deux substituants qui pris ensemble ne comptent pas plus de 4 atomes de carbone au total et qui sont identiques ou différents et sont choisis parmi halo; cyano; $C_{1-3}$ alcoyle ou $C_{1-3}$ alcoxy chacun facultativement substitué par hydroxyle ou $C_{1-2}$ alcoxy; $C_{1-2}$ alcoyle ou $C_{1-2}$ alcoxy halo-substitué; un radical $S(O)_nR^5$ où n représente 0, 1 ou 2 et $R^5$ représente $C_{1-2}$ alcoyle éventuellement substitué par hydroxyle ou $C_{1-2}$ alcoxy; ou bien Ar est éventuellement substitué par un radical $NR^6R^7$ ne comptant pas plus de 5 atomes de carbone, où $R^6$ et $R^7$ sont identiques ou différents et représentent chacun un radical $C_{1-3}$ alcoyle ou bien $NR^6R^7$ forme un radical hétérocyclique de cinq ou six chaînons contentant éventuellement un ou deux hétéroatomes supplémentaires;

$R^1$ représente $C_{1-3}$ alcoyle substituè par hydroxyle;

$R^2$ représente hydrogène, $C_{1-3}$ alcoyle ou hydroxyméthyle;

$R^3$ et $R^4$ sont identiques ou différents et représent chacun hydrogène, méthyle ou éthyle;

$R^1$, $R^2$, $R^3$ et $R^4$ pris ensemble ne comptant pas plus de 5 atomes de carbone;

ou bien:

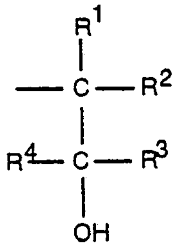

représente

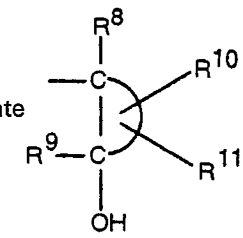

où

où 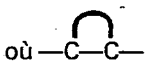 représente un cycle carbocyclique saturé de cinq ou six chaînons contenant deux ou trois radicaux hydroxyle;

$R^8$ représente hydrogène, méthyle ou hydroxyméthyle;

$R^9$ et $R^{10}$ sont identiques ou différents et représentent chacun hydrogène ou méthyle;

$R^{11}$ représente hydrogène, hydroxyle, méthyle ou hydroxyméthyle;

$R^8$, $R^9$, $R^{10}$, $R^{11}$ et le cycle 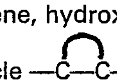 pris ensemble ne comptant pas plus de 7 atomes de carbone.

2. Composé suivant la revendication 1, dans lequel Ar représente 6-chrysényle, 7-fluoranthényle ou 1- ou 9-anthracényle substitué.

3. Composé suivant la revendication 1 ou 2, dans lequel Ar est substitué par $C_{1-2}$ alcoyle ou $C_{1-2}$ alcoxy chacun éventuellement substitué par chloro, hydroxyle ou méthoxy; ou un radical $S(O)_nR^5$ ou chloro, imidazolyle, morpholino, cyano ou bromo.

4. Composé suivant l'une quelconque des revendications 1 à 3, dans lequel

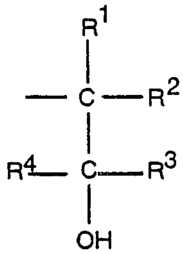

représente

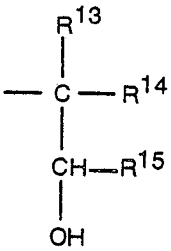

ou

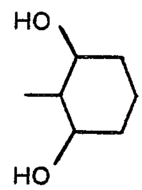

où

$R^{13}$ représente $CH_2OH$, $CH(CH_3)OH$ ou $CH_2CH_2OH$;

$R^{14}$ représente hydrogène, $C_{1-3}$ alcoyle, ou $CH_2OH$;

$R^{15}$ représente hydrogène ou méthyle.

5. Composé suivant l'une quelconque des revendications 1 à 4, dans lequel

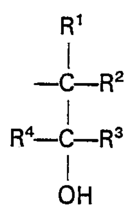

représente

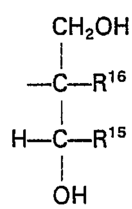

où $R^{15}$ représente hydrogène ou méthyle et $R^{16}$ représente hydrogène, méthyle ou éthyle.

6. Composé de formule (I) suivant la revendication 1, choisi parmi:

le 2-((6-chrysénylméthyl)amino)-2-méthyl-1,3-propanediol,
le 2-((9-anthracénylméthyl)amino)-2-méthyl-1,3-propanediol,
le 2-((1-anthracénylméthyl)amino)-2-méthyl-1,3-propanediol,
le 2-((10-chloro-9-anthracénylméthyl)-amino)-2-méthyl-1,3-propanediol,
le 2-((10-bromo-9-anthracénylméthyl)amino)-2-méthyl-1,3-propanediol,
le 2-méthyl-2-((10-méthyl-9-anthracénylméthyl)amino)-1,3-propanediol,
le 2-méthyl-2-((10-méthylthio-9-anthracénylméthyl)amino)-1,3-propanediol,
le 2-((10-(2-chloroéthyl)-9-anthracénylméthyl)amino)-2-méthyl-1,3-propanediol,
le 2-((10-hydroxyméthyl)-9-anthracénylméthyl)amino)-2-méthyl-1,3-propanediol,
le 10-((1,1-bis(hydroxyméthyl)éthylamino)méthyl-9-anthracéne-carbonitrile,
le 2-méthyl-2-((10-méthylsulfinyl-9-anthracénylméthyl)amino)-1,3-propanediol,
le 2-((10-méthoxy-9-anthracénylméthyl)amino)-2-méthyl-1,3-propanediol,
le 2-((10-bromo-1-anthracénylméthyl)amino)-2-méthyl-1,3-propanediol,

le 2-((4,10-dichloro-9-anthracénylméthyl)amino)-2-méthyl-1,3-propanediol,
le 2-((4,5-dichloro-9-anthracénylméthyl)amino)-2-méthyl-1,3-propanediol,
le 2-((2,10-dichloro-9-anthracénylméthyl)amino)-2-méthyl-1,3-propanediol,
le 2-((3,10-dichloro-9-anthracénylméthyl)amino)-2-méthyl-1,3-propanediol,
le 2-méthyl-2-((2-triphénylénylméthyl)amino)-1,3-propanediol,
le 2-((4-chloro-9-anthracénylméthyl)amino)-2-méthyl-1,3-propanediol,
le 2-((2-chloro-9-anthracénylméthyl)amino)-2-méthyl-1,3-propanediol,
le 2-((10-éthylthio-9-anthracénylméthyl)amino)-2-méthyl-1,3-propanediol,
le 2-((10-(2-hydroxyéthylthio)-9-anthracénylméthyl)amino)-2-méthyl-1,3-propanediol,
le 2-((10-chloro-9-anthracénylméthyl)amino)-2-hydroxyméthyl-1,3-propanediol,
le 2-((7-fluoranthénylméthyl)amino)-2-méthyl-1,3-propanediol,
le 2-((10-(2-hydroxyéthyloxy)-9-anthracénylméthyl)amino-2-méthyl-1,3-propanediol,
le 2-((10-éthoxy-9-anthracénylméthyl)amino)-2-méthyl-1,3-propanediol,
le 2-((6-chrysénylméthyl)amino)-2-hydroxyméthyl-1,3-propanediol,
le 2-((6-chrysénylméthyl)amino)-2-éthyl-1,3-propanediol,
le 2-hydroxyméthyl-2-((3-fluoranthénylméthyl)amino)-1,3-propanediol,
le 2-éthyl-2-((3-fluoranthénylméthyl)amino)-1,3-propanediol,
le 2-((10-chloro-9-anthracénylméthyl)amino)-2-éthyl-1,3-propanediol,
le 2-((3-chloro-9-anthracénylméthyl)amino)-2-méthyl-1,3-propanediol,
le (+ −)-(2R*,3S*)-2-((6-chrysénylméthyl)amino)-2-méthyl-1,3-butanediol,
le 2-((2-éthyl-9-anthracénylméthyl)amino)-2-méthyl-1,3-propanediol et
le 2-((3-éthyl-9-anthracénylméthyl)amino)-2-méthyl-1,3-propanediol,
le (+ −)(2R*,3S*)-2-((9-anthracénylméthyl)amino)-2-méthyl-1,3-butanediol,
le (+ −)(2R*,3R*)-2-(((6-chrysényl)méthyl)amino)-2-méthyl-1,3-butanediol,
le 2-(((6-chrysényl)méthyl)amino)-2-éthoxyméthyl-1,3-propanediol,
le 3-méthoxy-2-(((6-chrysényl)méthyl)amino)-2-méthyl-1-propanol,
le 3-méthoxy-2(((3-fluoranthényl)méthyl)amino)-2-méthyl-1-propanol,
le (+ −)(2R*,2S*)-2-(((3-fluoranthényl)méthyl)amino)-2-méthyl-1,3-butanediol,
le 2-éthoxyméthyl-2-(((3-fluoranthényl)méthyl)amino)-1,3-propanediol,
le 2-(((9-anthracényl)méthyl)amino)-2-éthoxyméthyl-1,3-propanediol,
le 2-β-((6-chrysénylméthyl)amino)-1-α,3-α-cyclohexanediol,
le 2-β-((3-fluoranthénylméthyl)amino)-1-α,3-α-cyclohexanediol,
le 2-((6-chrysénylméthyl)amino)-2-isopropyl-1,3-propanediol,
le 2-((3-fluoranthénylméthyl)amino)-2-isopropyl-1,3-propanediol,
le 2-((6-chrysénylméthyl)amino)-2-méthyl-1,4-butanediol,
le 2-((3-fluoranthénylméthyl)amino)-2-méthyl-1,4-butanediol,
le 2-(((10-chloro-1-anthracényl)méthyl)amino)-2-méthyl-1,3-propanediol,
le méso-3-((6-chrysénylméthyl)amino)-2,4-pentanediol,
le 2-((6-chrysénylméthyl)amino)-1,3-propanediol,
le 2-(((12-éthyl-6-chrysényl)méthyl)amino)-2-méthyl-1,3-propanediol,
le 2-(((10-(2-méthoxyéthoxy)-9-anthracényl)méthyl)amino)-2-méthyl-1,3-propanediol,
le 2-méthyl-2-(((10-morpholino-9-anthracényl)méthyl)amino)-1,3-propanediol,
le 2-((9-anthracénylméthyl)amino)-3-méthoxy-2-méthyl-1-propanol,
le 2-(((12-chloro-6-chrysényl)méthyl)amino)-2-méthyl-1,3-propanediol,
le 2-((9-anthracénylméthyl)amino)-2-isopropyl-1,3-propanediol,
le 2-((9-anthracénylméthyl)amino)-2-méthyl-1,4-butanediol,
le 2-(((10-(1*H*-imidazol-1-yl)-9-anthracényl)méthyl)amino)-2-méthyl-1,3-propanediol,
le 2-(4-éthyl-3-fluoranthényl)méthyl)amino)-2-méthyl-1,3-propanediol,
le 2-(((12-éthoxy-6-chrysényl)méthyl)amino)-2-méthyl-1,3-propanediol,
le (1α,2β,3α)-2-(9-anthracénylméthyl)amino)-1,3-cyclohexanediol
le 2-(((4-chloro-10-hydroxyéthoxy)-9-anthracényl)méthyl)amino)-2-méthyl-1,3-propanediol,
le (+ −)(2R$^+$,/RS$^+$, 4R$^+$)-3-(6-chrysénylméthyl)amino)-3-méthyl-2,5-pentanediol,
le 2-méthyl-2-(((10-méthylsulphonyl-1-9-anthracényl)méthyl)amino)-1,3-propanediol,
et leurs sels et esters.

7. Le 2-((6-chrysénylméthyl)amino)-2-méthyl-1,3-propanediol ou un sel ou ester de celui-ci.

8. Chlorhydrate, méthansulfonate, éthanesulfonate, lactate, citrate ou iséthionate salin d'un composé de formule (I) suivant l'une quelconque des revendications 1 à 8.

9. Composition pharmaceutique comprenant un composé de formule (I) ou un éther, ester ou sel de celui-ci suivant l'une quelconque des revendications 1 à 8, conjointement avev un excipient pharmaceutiquement acceptable.

10. Composition pharmaceutique suivant la revendication 9, qui comprend une solution aqueuse stérile d'un sel d'addition d'acide pharmaceutiquement et pharmacologique acceptable d'un composé de formule (I) suivant l'une quelconque des revendications 1 à 6, qui est isotonique à l'égard du sang du receveur.

11. Porcéde de préparation d'une composition pharmaceutique, qui comprend la mise en association

d'un composé de formule (I) ou d'un éther, ester ou sel pharmaceutiquement acceptable de celui-ci avec un excipient pharmaceutiquement acceptable.

12. Procédé de préparation d'un composé de formule (I) l'une quelconque des revendications 1 à 7, qui comprend:

(i) la réduction d'un composé de formule (II):

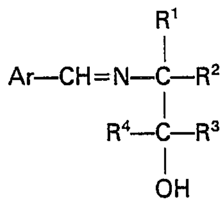

(II)

où $R^1$ à $R^4$ sont tels que définis ci-dessus, ou d'un dérivé convenablement protégé de celui-ci, suivie de la déprotection le cas échéant;

(ii) la réduction d'un composé de formule (V):

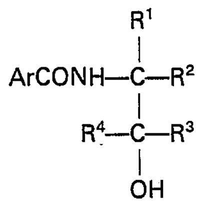

(V)

ouì $R^1$ à $R^4$ sont tels que définis ci-dessus et les radicaux hydroxyle sone éventuellement protégés, suivie de la déprotection des radicaux hydroxyle le cas échéant, ou

(iii) la réaction d'un composé $ArCH_2L$, où Ar est tel que défini ci-dessus et L est un radical partant, avec un composé de formule (IV):

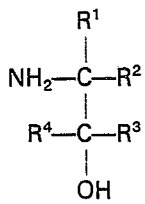

où $R^1$ à $R^4$ sont tels que définis ci-dessus.

13. Intermédiaire chimique de formule (II) ci-après:

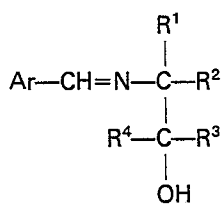

(II)

où Ar, $R^1$, $R^2$, $R^3$ et $R^4$ sont tels que définis dans la revendication 1.

14. Intermédiaire chimique de formule (V) ci-après:

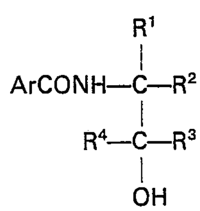

où Ar, $R^1$, $R^2$, $R^3$, et $R^4$ sont tels que définis dans la revendication 1.

15. Le 2(((10-(2-hydroxyéthoxy)-9-anthracényl)méthyl)amino)-2-méthyl-1,3-propanediol ou un sel ou ester de celui-ci.

16. Le 2((3-fluroanthénylméthyl)amino)-2-méthyl-1,3-propanediol ou un sel ou ester de celui-ci.